# FASCICULE DE BREVET EUROPEEN

(11) **EP 1 601 346 B1**
(45) Date de publication et mention de la délivrance du brevet: **12.08.2009**
(21) Numéro de dépôt: 04715360.6
(22) Date de dépôt: 27.02.2004
(51) Int. Cl.: A61K 9/14, A61K 31/216, A61K 9/20, A61K 31/505, A61K 31/70, A61K 31/195, A61K 31/365, A61K 31/40, A61K 31/405

(54) **COMPRIME DE FENOFIBRATE ET PROCEDE DE FABRICATION**
FENOFIBRATE TABLETT UND VERFAHREN ZUR HERSTELLUNG
METHOD FOR PRODUCING A PHARMACEUTICAL COMPOSITION IN THE FORM OF FIBRATE-CONTAINING TABLETS AND TABLETS PRODUCED BY SAID METHOD

(30) Priorité: 28.02.2003 FR 0302520
(43) Date de publication de la demande: 07.12.2005
(73) Titulaire: SARL Galenix Innovations, 33127 Saint-Jean D'Illac (FR)
(72) Inventeur: BESSE, Jérôme, F-33480 Listrac Medoc (FR)
(74) Mandataire: Augarde, Eric
(86) Numéro de dépôt international: PCT/FR2004/050090
(87) Numéro de publication internationale: WO 2004/078108

(56) Documents cités:
- EP-A- 0 330 532
- EP-A- 0 724 877
- FR-A- 2 758 461
- FR-A- 2 819 720

## Description

La présente invention se rapporte au domaine de la fabrication de nouvelles formules galéniques contenant le principe actif fénofibrate ou l'un de ses dérivés, éventuellement sous la forme d'une association avec un second principe actif.

Le 2-[4-(4-chlorobenzyl)phénoxy]-2-méthylpropionate d'isopropyle, dont la dénomination commune internationale (DCI) est fénofibrate, est un principe actif hypolipidémiant. Le fénofibrate est le principe actif le plus utilisé dans le monde pour le traitement des hypercholestérolémies et des hypertriglycéridémies endogènes de l'adulte, isolées ou associées. Son efficacité dans ces indications thérapeutiques a été largement démontrée. Ainsi, administré à long terme à des doses thérapeutiquement efficaces, le fénofibrate permet d'abaisser la cholestérolémie de 20 à 25% et la triglycéridémie de 40 à 50% et ce, dès le premier mois de traitement.

Afin d'obtenir un effet hypocholestérolémiant satisfaisant, il est souhaitable de maintenir un taux plasmatique (circulant) d'acide fénofibrique, qui est le métabolite actif du fénofibrate, dans une gamme de concentrations plasmatiques comprises entre environ 6 et environ 10 mg/l. Un traitement de longue durée par le fénofibrate n'est toutefois pas totalement dénué d'effets indésirables. On a notamment rapporté des effets indésirables tels que la rhadomyolyse, l'asthénie, une augmentation de l'urée sanguine, une augmentation de la créatininémie, des éruptions cutanées, de l'urticaire, des nausées, des vomissements, des douleurs abdominales, des diarrhées, une augmentation des transaminases, une toxicité hépatique, une diminution des phosphatases alcalines, une polymyosite, des douleurs musculaires, des céphalées et des vertiges.

C'est la raison pour laquelle des travaux ont été réalisés dans le but de développer des formulations galéniques permettant une biodisponibilité accrue du principe actif fénofibrate lorsque celui-ci est absorbé par la voie orale, de manière à diminuer la dose de fénofibrate et ainsi à diminuer, par voie de conséquence, les risques d'effets indésirables.

Le fénofibrate est un principe actif très faiblement soluble dans l'eau dont l'absorption au niveau du tractus digestif est limité. Ces propriétés de faible solubilité dans l'eau du fénofibrate ont été prises en compte pour la conception et la réalisation des diverses formulations pharmaceutiques de l'état de la technique contenant ce principe actif.

Ainsi, diverses voies ont été explorées pour augmenter la vitesse de solubilisation du fénofibrate et pour obtenir des compositions pharmaceutiques aisément administrables afin d'améliorer le confort du patient traité.

Une première solution technique a consisté à microniser le fénofibrate en association avec un agent tensioactif, comme cela est décrit dans la demande de brevet européen N°EP 330 532.

La fabrication de gélules contenant des granules d'un co-micronisat de fénofibrate avec un excipient, mais en l'absence de surfactant, a aussi été décrit dans l'état de la technique, comme par exemple dans la demande de brevet européen n°EP 1 048.295.

La principale forme pharmaceutique dans laquelle ont été commercialisées des spécialités pharmaceutiques à base de fénofibrate a consisté en des gélules, en particulier des gélules de gélatine molle ou des gélules de gélatine dure contenant des granules d'un support constitué d'excipients pharmaceutiques en association avec le fénofibrate.

Un exemple de gélule contenant des granules constitués d'un support d'excipient pharmaceutique en association avec un co-micronisat de fénofibrate avec un agent tensioactif est décrit dans le brevet américain N°US 4,895,726. Pour la préparation des granules contenus dans de telles gélules, et en vue de surmonter les problèmes liés à la faible dissolution dans l'eau du fénofibrate, entraînant une biodisponibilité réduite de ce principe actif, il a été mis en oeuvre des procédés de préparation dans lesquels le fénofibrate est mis en suspension dans une solution de polymère hydrophile, puis la suspension est pulvérisée sur un support inerte d'excipients hydrodispersibles, comme cela est décrit dans la demande de brevet français publiée sous le n°2.758.459. Un tel procédé a été amélioré par l'incorporation d'un dérivé cellulosique utilisé comme liant et adjuvant de solubilisation dans la suspension aqueuse destinée à être pulvérisée sur le support d'excipients inertes, comme cela est décrit dans la demande PCT publiée sous le n°WO 01/03.693.

Dans d'autres formulations pharmaceutiques sous forme de gélules, d'autres solutions techniques destinées à améliorer la biodisponibilité du fénofibrate ont été mises au point, comme (i) la granulation du fénofibrate en présence d'un milieu liquide comprenant un agent tensioactif, de l'eau et un alcool hydromiscible, comme décrit dans la demande de brevet français publiée sous le n°FR 2.783.421, (ii) la réalisation d'un préconcentré capable de former spontanément une microémulsion huile-dans-eau au contact d'un milieu aqueux, ledit préconcentré comprenant une phase lipophile et un système émulsifiant, comme cela est décrit dans la demande de brevet français publiée sous le n°2.803.203, (iii) la réalisation d'une association entre le fénofibrate micronisé et un phospholipide, comme cela est décrit dans la demande de brevet américain publiée sous le n°US 2002/0119199, et (iv) la réalisation d'une suspension de fénofibrate en solution dans l'éther monoéthylique du diéthylèneglycol (EMDG), comme cela est décrit dans la demande de brevet européen N°EP 0757 911.

On a réalisé aussi des formulations pharmaceutiques à base de fénofibrate sous la forme de comprimés. Des formulations pharmaceutiques à base de fénofibrate sous forme de comprimés présentent l'avantage d'être plus petites, à poids égal, qu'une gélule et permettent, en outre, une production industrielle de telles formulations pharmaceutiques à des cadences supérieures à celles observées pour la fabrication des formulations sous forme de capsules molles ou de gélules. Pour la fabrication de tels comprimés, et compte tenu de la faible hydrosolubilité du fénofibrate, il est d'abord préparé une suspension de fénofibrate dispersé dans une solution aqueuse d'un polymère hydrophile, telle que la polyvinylpyrrolidone (PVP). Dans un second temps, soit (i) la solution aqueuse de polymère hydrophile contenant le fénofibrate est pulvérisée sur un support inerte d'excipients, comme cela est décrit dans la demande de brevet européen N°EP 1.273.293 ou encore dans la demande de brevet français N°FR 2.758.461, soit (ii) la suspension de fénofibrate, éventuellement co-micronisée avec un agent tensioactif, est granulée par granulation liquide dans une solution aqueuse de polyvinylpyrrolidone, avant incorporation d'éventuels autres excipients, préalablement à une étape de compression par voie humide pour l'obtention finale d'un comprimé, comme cela est décrit dans la demande de brevet français n°FR 2 819 720.

Comme on le voit, on a cherché, dans l'état de la technique, à améliorer progressivement les techniques de solubilisation du principe actif fénofibrate en vue d'accroître sa biodisponibilité vis-à-vis des sites cibles dans l'organisme, y compris par la fabrication de formulations pharmaceutiques sous forme de comprimés, ces derniers présentant de nombreux avantages techniques à la fois du point de vue des conditions de leur production industrielle et du point de vue du confort pour le patient à traiter.

Il existe un besoin dans l'état de la technique d'améliorer encore les caractéristiques techniques d'une composition pharmaceutique à base d'un principe actif du type fibrate, en particulier le fénofibrate, et il est encore souhaitable aujourd'hui (i) d'améliorer les conditions industrielles de production de telles formulations pharmaceutiques et (ii) de fabriquer de telles formulations pharmaceutiques permettant l'obtention d'une biodisponibilité du fibrate *in vivo,* au moins égale à celle des compositions connues afin d'obtenir une bonne efficacité thérapeutique du traitement et également de minimiser les effets indésirables de ce principe actif.

Les objectifs ci-dessus ont été atteints selon la présente invention.

De manière surprenante, il est montré selon l'invention qu'une formulation pharmaceutique à base d'un principe actif du type fibrate, sous la forme de comprimés, peut être obtenue sans nécessiter de dispersion ou solubilisation du fibrate dans une solution aqueuse contenant un polymère hydrophile, préalablement à la préparation du support pharmaceutique solide destiné à être compressé sous la forme de comprimés.

Ainsi, il est montré selon l'invention qu'une composition pharmaceutique contenant un fibrate, qui est préparé sans étape de solubilisation du fibrate en solution aqueuse, présente une biodisponibilité *in vivo* du principe actif et des propriétés pharmacocinétiques au moins équivalentes à celles qui sont observées avec les compositions pharmaceutiques sous forme de comprimés obtenues par granulation humide connues dans l'état de la technique.

Il a donc été mis au point selon l'invention un procédé de fabrication d'une composition pharmaceutique de comprimés à base de fibrate, qui comprend exclusivement des étapes de granulation ou de compactage qui sont réalisées par voie sèche.

L'invention a pour objet un procédé pour la fabrication d'une composition pharmaceutique contenant le principe actif fénofibrate ou l'un de ses dérivés sous la forme de comprimés, caractérisé en ce qu'il comprend les étapes suivantes :
(a) préparer un mélange de fénofibrate ou l'un de ses dérivés, éventuellement sous la forme d'une association du fénofibrate ou de son dérivé avec un second principe actif, et d'au moins un agent tensioactif solide, dans une proportion de (i) 91 % à 99 % en poids de fénofibrate ou de son dérivé, ou de l'association avec un second principe actif, et (ii) 1 % à 9 % en poids dudit ou desdits agent(s) tensioactif(s) solide(s);
(b) microniser le mélange de fénofibrate ou l'un de ses dérivés, éventuellement sous la forme d'une association avec un second principe actif, et d'agent(s) tensioactif(s) obtenu à l'étape (a), afin d'obtenir un micronisat du fénofibrate, ou de l'association avec le second principe actif, et du ou des agent(s) tensioactif(s) ;
(c) additionner au moins un agent anti-statique au micronisat préparé à l'étape (b);
(d) additionner au mélange obtenu à l'étape (c) au moins un agent diluant, au moins un agent désintégrant et au moins un agent lubrifiant, afin d'obtenir un mélange solide correspondant à la phase interne du comprimé ;
(e) compacter, selon une étape de granulation par voie sèche, le mélange solide obtenu à l'étape (d) afin d'obtenir la phase interne finale du comprimé;
(f) mélanger la phase interne du comprimé préparée à l'étape (e) avec une phase externe comprenant au moins un agent lubrifiant, puis effectuer une compression de la composition afin d'obtenir la composition pharmaceutique contenant le fénofibrate sous la forme de comprimés.

Un dérivé de fénofibrate selon l'invention englobe tout type de principe actif du type fibrate, autre que le fénofibrate, y compris l'acide 2-[4-[2-chlorobenzamido)éthyl]phénoxy]-2-méthylpropionique (Bezafibrate), l'acide 2-[4-(2,2-dichlorocyclopropyl)phénoxy]-2-méthylpropionique (Ciprofibrate), le 2-(4-chlorophénoxy)-2-méthylpropionate de 3-diméthylcarbamoylpropyle (Clofibride), le 2bis-(4-chlorophénoxy)-2-méthylpropionate d'hydroxyaluminium (Clofibrate d'aluminium) et l'acide 2,2-diméthyl-5-(2,5-xylyloxy)valérique (Gemfibrozil). Tous les principes actifs du type fibrate possèdent en commun une propriété pharmacologique d'hypolipidémiant et une propriété physico-chimique de faible solubilité dans un milieu aqueux.

Dans une composition pharmaceutique préparée conformément à l'invention, le fénofibrate ou l'un de ses dérivés peut être contenu sous la forme d'une association avec un second principe actif, tel que la metformine, comme décrit dans la demande PCT N° WO 99/40904, la cobalamine, l'acide folique, la bétaïne ou la N-acétylcystéine comme décrit dans le brevet allemand N°DE 1.991.0682, la vitamine E, comme décrit dans la demande de brevet français n°FR 19 95 000 126, un inhibiteur de HMG CoA Cstatine, comme décrit dans la demande PCT N°WO 01 37 831 ou encore dans la demande PCT N° WO 02 34 359.

Le procédé ci-dessus, qui ne comprend aucune étape de solubilisation du fénofibrate ou de son dérivé dans une solution aqueuse d'un polymère hydrophile, ni, par voie de conséquence, d'étape de granulation humide préalable à la fabrication du comprimé final, est donc considérablement simplifié dans sa mise en oeuvre, vis-à-vis des procédés antérieurement connus. La mise en oeuvre à l'échelle industrielle du procédé selon l'invention permet donc (i) une économie de moyens, du fait de l'absence d'une étape de solubilisation du fénofibrate ou de son dérivé et (ii) un plus haut rendement, du fait de sa simplicité de mise en oeuvre permettant d'accroître la cadence de production industrielle des comprimés, par rapport aux procédés connus dans l'état de la technique. De plus, le procédé selon l'invention, du fait qu'il ne comprend pas d'étape de solubilisation du fénofibrate ou de son dérivé dans une suspension aqueuse, par exemple dans une suspension aqueuse contenant un polymère hydrophile, et qu'en conséquence l'ensemble des étapes dudit procédé est réalisé exclusivement par voie sèche, peut être réalisé en continu, ce qui en augmente le rendement et en diminue le coût.

De plus, comme cela est illustré dans les exemples, les comprimés à base de fibrate obtenus selon le procédé de l'invention possèdent des propriétés pharmacologiques au moins identiques, à dosage égal de fibrate, par rapport aux comprimés ou aux gélules de fibrate connus antérieurement.

De préférence, à l'étape (a) du procédé, on a introduit le fénofibrate ou son dérivé et l'agent tensioactif dans un dispositif mélangeur, par exemple dans un dispositif mélangeur de type Turbula®, ou équivalent puis on procède au mélange du principe actif et du ou des tensioactifs. Avantageusement, le mélange des deux types de composés est réalisé pendant une durée de dix minutes, par exemple à une vitesse de 50 tours/min.

Dans un mode de réalisation particulier de l'étape (a), le fénofibrate ou son dérivé se présente sous la forme d'une association avec un second principe actif et on introduit le mélange du fénofibrate ou de son dérivé avec le second principe actif et l'agent tensioactif dans le dispositif mélangeur.

L'étape (b) du procédé peut être réalisée conformément à l'enseignement de la demande de brevet européen n°EP 0330 532.

De préférence, l'étape (b) du procédé est réalisée par une micronisation du fénofibrate ou de son dérivé, éventuellement sous la forme d'une association avec un second principe actif, et du ou des agents tensioactifs par la méthode conventionnelle à jet d'air, par exemple en utilisant un appareil de micronisation à jet d'air de type ALPINE ou JET MILL, selon les recommandations du fabricant.

Les paramètres préférés pour une micronisation sur un appareil microniseur GALETTE ALPINE 200 AS sont les suivants :
- injecteur : 7 à 8 bars ;
- couronne : 4 à 6 bars ;
- débit d'air : 180 m³/H ; et
- vitesse : 25 kg/h.

Les conditions ci-dessus de l'étape (b) de micronisation du fibrate, éventuellement sous la forme d'une association avec un second principe actif, et du ou des agents tensioactifs permet l'obtention d'un co-micronisat comprenant les particules possédant une taille comprise entre 0,1 µm et 20µm.

En général, environ 90% des particules du micronisat ont une taille comprise entre environ 0,8 µm et environ 7 µm. Selon les essais, la taille moyenne des particules du micronisat est comprise entre 2,5 µm et 7µm.

La taille moyenne des grains ou particules du micronisat peut être mesurée par toute technique conventionnelle connue en soi. Notamment, l'homme du métier peut avoir recours à une mesure de la granulométrie à laser avec un dispositif du type Beckman Coulter ou Malvern, comme cela est décrit dans les exemples.

Avantageusement, l'étape (c) du procédé est réalisée en introduisant le micronisat obtenu à l'étape (b) et le ou les agents antistatiques dans un conteneur, réaliser un mélange, puis tamiser ce mélange, par exemple à l'aide d'un tamis ayant une taille de maille de 500 µm. Puis, le mélange tamisé ainsi obtenu est introduit dans la cuve d'un mélangeur, par exemple du type Bohle®

Puis, à l'étape (d) du procédé, on additionne au mélange obtenu à l'étape (c) au moins un agent diluant et au moins un agent désintégrant, puis on procède à un mélange à l'aide dudit dispositif mélangeur, par exemple pendant une durée d'environ vingt minutes.

Subséquemment, on introduit la quantité appropriée d'au moins un agent lubrifiant, éventuellement tamisé, par exemple avec un tamis ayant une taille de maille d'environ 500 µm, puis on procède à un nouveau mélange dans le même dispositif mélangeur, par exemple pendant une durée d'environ trois minutes.

A l'étape (e) du procédé, on réalise un compactage du mélange solide obtenu à l'étape (d) à l'aide de tout dispositif de compactage conventionnel, comme par exemple le dispositif de compactage Alexanderwerck®, qui peut être équipé d'une grille inox d'ouverture de maille de 1,25mm.

L'étape (e) de compactage confère des caractéristiques pharmacotechniques avantageuses au mélange avant compression, notamment en ce qui concerne les propriétés d'écoulement, sans que les profils de dissolution *in vitro* et *in vivo* du comprimé produit fini ne soient modifiés, par rapport aux profils de dissolution d'un comprimé comparatif obtenu par un procédé ne comportant pas l'étape (e) de compactage. L'étape de compactage a pour effet d'augmenter la densité du mélange solide obtenu à l'étape (d), qui est donc facilement utilisable par gravité au moment de la compression, à l'étape (f) du procédé.

Les étapes (a) à (e) du procédé permettent la fabrication de la phase interne du comprimé.

A l'étape (f) du procédé, on mélange la phase interne finale du comprimé préparée à l'étape (e) avec une phase externe comprenant au moins un agent lubrifiant. De préférence, l'étape (f) est réalisée en ajoutant la quantité appropriée du ou des agents lubrifiants, éventuellement préalablement tamisés, et on mélange la phase interne préparée à l'étape (e) avec ledit ou lesdits agents lubrifiants sur un dispositif mélangeur classique, par exemple un mélangeur de type Bolhe®, par exemple pendant une durée d'environ trois minutes.

A l'étape (f), le recouvrement de la surface de la phase interne finale du comprimé obtenu à l'étape (e) avec un ou plusieurs agent(s) lubrifiant(s) permet d'éviter le phénomène de grippage au moment de l'étape subséquente de compression de la composition sous forme de comprimés, c'est-à-dire de frottement entre les pièces du dispositif de compactage et la poudre à compacter, susceptible de provoquer un blocage de l'étape de compactage, qui est autrement observé en l'absence du revêtement de la phase interne finale par ledit agent lubrifiant.

A l'étape (f), la compression est réalisée sur tout dispositif conventionnel. Par exemple, l'homme du métier peut avoir recours à une machine à comprimer rotative du type Kilian®.

Pour la compression, on peut notamment utiliser les poinçons plats, ronds, ou oblongs y compris des poinçons de type 16R16, ou encore des poinçons courbes, ronds, y compris des poinçons type 9,5 R8 ou encore des poinçons de format oblong18 x 8R8.

Selon un premier aspect préféré de l'étape (a) du procédé de l'invention, le mélange de fénofibrate ou de son dérivé et d'agent(s) tensioactif(s) comprend une proportion de (i) 95 % à 98 % en poids de fénofibrate ou de son dérivé et (ii) 2 % à 5 % en poids d'agent(s) tensioactif(s).

Dans le mode de réalisation particulier du procédé dans lequel le fénofibrate ou son dérivé est sous la forme d'une association avec un second principe actif, le mélange comprend une proportion de (i) 95% à 98% en poids de l'association entre le fénofibrate ou son dérivé et le second principe actif et (ii) 2% à 5% en poids d'agent(s) tensioactif(s).

De manière tout à fait préférée, le mélange de fénofibrate ou de son dérivé, éventuellement sous la forme d'une association avec un second principe actif, et d'agent(s) tensioactif(s) comprend une proportion de 96,6% en poids de fénofibrate ou de son dérivé, ou de l'association, et 3,4% en poids dudit ou desdits agent(s) tensioactif(s).

Le ou les agent(s) tensioactif(s) est (sont) préférentiellement choisi(s) parmi les agents tensioactifs suivants : sodium lauryl sulfate, un ester de polysorbitanne polyoxyéthyléné, tel que les esters monooléate, monolaurate, monopalmitate, monostéarate, dioctylsulfosuccinate de sodium (DOSS) et lécithine.

De manière tout à fait préférée, l'agent tensioactif est le laurylsulfate de sodium.

Selon un premier aspect préféré de l'étape (b) du procédé, le micronisat comprend des particules possédant une taille comprise entre 0,1 et 20 µm.

De préférence, la taille moyenne des grains ou particules du micronisat est comprise entre 2 µm et 7 µm.

Selon un premier aspect préféré de l'étape (c) du procédé, le ou les agent(s) antistatique(s) est (sont) ajouté(s) à raison de 0,1 % à 5 % en poids, de préférence de 0,2 % à 2 % en poids, par rapport au poids total de la composition.

Le ou les agent(s) antistatique(s) augmentent les propriétés d'écoulement de la poudre et favorisent en conséquence la dispersion des différents constituants, y inclus le fenofibrate ou son dérivé, éventuellement sous la forme d'une association avec un second principe actif, de manière homogène dans le mélange, dans le but d'obtenir, à la fin du procédé, des comprimés contenant tous la quantité désirée du (des) principe(s) actif(s), et des différents excipients.

Préférentiellement, le ou les agent(s) antistatique(s) est (sont) ajouté(s) à raison de 0,4% à 0,7% en poids, par rapport au poids total de la composition.

De manière tout à fait préférée, le ou les agent(s) antistatique(s) est (sont) ajouté(s) à raison de 0,49% en poids ou de 0,50% en poids, par rapport au poids total de la composition.

Préférentiellement, le ou les agent(s) antistatique(s) est (sont) choisi(s) parmi la silice colloïdale, le silicate de magnésium, le talc, le silicate de calcium et le phosphate de calcium tribasique seul ou en association.

De manière tout à fait préférée on utilise un seul agent antistatique, qui est la silice colloïdale anhydre.

Selon un premier aspect préféré de l'étape (d) du procédé, le ou les agents diluant(s) est (sont) ajouté(s) à raison de 40 % à 80 % en poids, de préférence entre 50 % et 75 % en poids, par rapport au poids total de la composition.

Par agent diluant, on entend selon l'invention un agent utilisé pour compléter la composition pharmaceutique préparée selon le procédé, jusqu'à obtention d'un volume total prédéterminé contenant la quantité choisie de fénofibrate ou de son dérivé, ou de l'association du fénofibrate ou de son dérivé avec un second principe actif, le volume de fibrate ou de l'association étant insuffisant pour la réalisation d'une composition pharmaceutique finale dont le volume désiré comprend la quantité adaptée de ce principe actif.

De préférence, au moins l'un des agents diluants exerce aussi la fonction d'un agent liant, comme par exemple la cellulose microcristalline.

De manière tout à fait préférée, le ou les agent(s) diluant(s) est sont ajouté(s) à raison de 66% à 72% en poids, et encore mieux à raison de 68,5% à 70,5% en poids, par rapport au poids total de la composition. Les agents diluants préférentiellement utilisés pour la mise en oeuvre du procédé selon l'invention sont choisis parmi le carbonate ou bicarbonate de calcium ou de sodium, le sucrose, le mannitol, le xylitol, le sorbitol, le lactose, le maltitol, le glucose, la poudre de cellulose ou cellulose microcristalline, l'amidon et ses dérivés, le phosphate de calcium dibasique, le phosphate de calcium tribasique, le sulfate de calcium, les dextrates, les dextrines, les excipients de dextrose, le fructose, le kaolin, le lactitol.

De manière tout à fait préférée, on utilise une combinaison de deux agents diluants, respectivement la cellulose microcristalline et le lactose monohydraté.

Selon un second aspect préféré de l'étape (d) du procédé, le ou les agent(s) désintégrant(s) est (sont) ajouté(s) à raison de 1 % à 20 % en poids, de préférence de 3 à 6 % en poids, par rapport au poids total de la composition.

De manière tout à fait préférée, le ou les agent(s) désintégrant(s) est (sont) ajouté(s) à raison de 4,5% à 5,5% en poids, et encore mieux de 4,8% à 5,1% en poids, par rapport au poids total de la composition.

Préférentiellement, les agents désintégrants sont choisis parmi le glycolate d'amidon sodique, la croscarmellose sodique, la polyvinylpyrrolidone réticulée, la carboxyméthylcellulose de sodium et la carboxyméthylcellulose de calcium et l'hydroxypropylcellulose faiblement substituée.

De manière tout à fait préférée, on utilise, en tant qu'agent désintégrant, la croscarmellose sodique.

Selon un troisième aspect préféré de l'étape (d) du procédé, le ou les agent(s) lubrifiant(s) est (sont) ajouté(s) à raison de 0,1 % à 2 % en poids, de préférence de 0,2 % à 1 % en poids, par rapport au poids total de la composition.

De manière tout à fait préférée, le ou les agent(s) lubrifiant(s) est (sont) ajouté(s) à raison de 0,2% à 0,8% en poids, et encore mieux à raison de 0,5% en poids, par rapport au poids total de la composition.

Selon le procédé, le ou les agent(s) lubrifiant(s) est (sont) (choisi(s) parmi le stéarate de magnésium le stéarate de calcium et le talc.

De manière tout à fait préférée, on utilise, en tant qu'agent lubrifiant, le stéarate de magnésium.

Préférentiellement, on utilise une quantité de fénofibrate ou de son dérivé, éventuellement sous la forme d'une association avec un second principe actif, à l'étape (a) du procédé, telle que le fénofibrate ou de son dérivé, ou l'association du fénofibrate ou son dérivé avec le second principe actif, est présent à raison de 20% à 50% en poids, par rapport au poids total de la composition pharmaceutique finale obtenue à l'étape (f).

Préférentiellement, on utilise le fénofibrate ou son dérivé, éventuellement sous la forme d'une association avec un second principe acif, dans des quantités telles que le fénofibrate ou son dérivé, ou l'association avec le second principe actif, est présent à raison de 20 à 25% en poids, et encore mieux de 22% à 24% en poids, par rapport au poids total de la composition finale obtenue à l'étape (f) du procédé.

Selon un mode de réalisation particulier du procédé selon l'invention, ledit procédé comprend une étape supplémentaire (g) de pelliculage du comprimé qui a été obtenu à l'étape (f), la pellicule qui recouvre alors la totalité de la surface de la phase externe du comprimé obtenu à la fin de l'étape (f) permettant de protéger la composition de comprimés de l'environnement extérieur, pour une meilleure conservation. De plus, la pellicule permet de masquer l'amertume caractéristique du principe actif fénofibrate ou de ses dérivés. Les résultats présentés dans les exemples montrent que le pelliculage du comprimé tel qu'obtenu à la fin de l'étape (f) du procédé n'entraîne pas de modification détectable du profil de libération du fénofibrate.

Toutefois, ce pelliculage n'est pas indispensable, la bonne conservation du comprimé pouvant être assurée par d'autres moyens, comme par exemple par un conditionnement limitant les échanges de vapeur d'eau avec l'extérieur.

Le pelliculage peut être réalisé de façon classique, selon des procédés connus de l'homme de l'art, par exemple par pulvérisation d'une solution de polymère filmogène sur les comprimés placés dans une turbine. Ce pelliculage permet également, le cas échéant, de colorer les comprimés par ajout d'un pigment coloré à la solution de polymère de pelliculage.

Pour le pelliculage du comprimé obtenu à la fin de l'étape (a) du procédé, l'homme du métier peut notamment utiliser un dérivé cellulosique, comme l'hydroxypropylméthylcellulose, par exemple l'hydroxypropylméthylcellulose commercialisée sous la dénomination Opadry®, qui peut être mis en suspension à la concentration de 15% en poids, par rapport au poids de la solution aqueuse utilisée pour le pelliculage.

Un pelliculage préféré selon l'invention comprend (i) un agent filmogène, tel que l'hydroxypropylméthylcellulose, (ii) un agent plastifiant, tel que le polyéthylène glycol, (iii) un agent diluant, tel que le lactose et (iv) d'un agent de charge, qui peut avoir une fonction d'opacifiant, tel que le dioxyde de titane.

On a montré selon l'invention que le procédé tel que décrit ci-dessus permet l'obtention facilement, et à moindre coût, de comprimés de fénofibrate ou de l'un de ses dérivés, éventuellement en association avec un second principe actif, lesquels possèdent un profil pharmacocinétique *in vivo* au moins identique à celui observé avec les comprimés de fénofibrate préparés avec un procédé incluant une étape de granulation humide, comme dans l'état de la technique.

Les valeurs de concentration plasmatique maximum (Cmax), de temps nécessaire à l'obtention de la concentration plasmatique maximum (Tₘₐₓ) et d'aire sous la courbe de concentration plasmatique (ASC) ont été calculées à partir des profils pharmacocinétiques, pour des comprimés préparés selon l'invention et pour des gélules préparées avec un procédé incluant une étape de granulation humide, comme dans l'état de la technique. L'ensemble de ces valeurs a été comparé dans les exemples 3 et 4.

L'invention a donc également pour objet un comprimé de fénofibrate ou de l'un de ses dérivés, susceptible d'être obtenu par le présent procédé caractérisé en ce qu'il possède une concentration plasmatique maximum équivalente à celle d'un comprimé préparé avec un procédé incluant une étape de granulation humide.

L'invention a encore pour objet un comprimé de fénofibrate ou de l'un de ses dérivés, susceptible d'être obtenu par le présent procédé caractérisé en ce qu'il possède une aire sous la courbe de concentration plasmatique (ASC) équivalente à celle que l'on peut mesurer pour un comprimé préparé avec un procédé incluant une étape de granulation humide, ou pour une gélule.

Par le terme « équivalent» l'homme du métier comprendra des valeurs moyennes non statistiquement différentes.

Le procédé de l'invention est adapté notamment à la fabrication de comprimés comprenant une concentration en fénofibrate comprise entre 30 mg et 300 mg.

De préférence, le comprimé de l'invention est dosé à 200 mg, et possède un profil pharmacocinétique caractérisé par une aire sous la courbe de concentration plasmatique mesurée *in vivo* (ASC), de l'ordre de 220000 ng.h/ml.

De préférence, le comprimé de l'invention est dosé à 200 mg, et possède un profil pharmacocinétique caractérisé par une valeur de concentration plasmatique maximum (Cₘₐₓ) de l'ordre de 10600 ng/ml.

De préférence, le comprimé de l'invention est dosé à 200 mg, et possède un profil pharmacocinétique caractérisé par une valeur de Tₘₐₓ comprise entre 2,00 et 3,75 ng/ml.

Par « valeur de Tₘₐₓ », l'homme du métier comprendra le temps nécessaire à l'obtention de la concentration plasmatique maximum.

L'invention a encore pour objet un comprimé de fénofibrate ou de l'un de ses dérivés, susceptible d'être obtenu par le présent procédé caractérisé en ce que :
(a) une phase interne constituée de :
   (i) de 20 % à 50 % en poids de fénofibrate ou de son dérivé, éventuellement sous la forme d'une association avec un second principe actif, sous la forme d'un micronisat avec au moins un agent tensioactif solide, ledit micronisat comprenant (i) de 91 % à 99 % en poids de fénofibrate ou de son dérivé, éventuellement sous la forme d'une association avec un second principe actif, et (ii) de 1 % à 9 % en poids dudit ou desdits agent(s) tensioactif(s) solide(s);
   (ii) de 0,2 % à 2 % en poids d'au moins un agent antistatique ;
   (iii) de 40 % à 80 % en poids d'au moins un agent diluant ;
   (iv) de 1 % à 20 % en poids d'au moins un agent désintégrant ; et
   (v) de 0,1 % à 1 % d'au moins un agent lubrifiant ;
      par rapport au poids total dudit comprimé ; et
(b) une phase externe comprenant de 0,1 % à 2 % d'au moins un agent lubrifiant, par rapport au poids total dudit comprimé.

Selon un mode de réalisation avantageux, ledit comprimé est caractérisé en ce que la phase externe est recouverte d'un vernis protecteur, de préférence un vernis protecteur à base d'un polymère hydrodispersible.

Comme illustré dans les exemples, diverses formulations pharmaceutiques sous forme de comprimés contenant le principe actif fénofibrate ou l'un de ses dérivés, et préparées conformément au procédé de l'invention, ont été réalisées. Ces compositions pharmaceutiques spécifiques font également partie de l'invention.

L'invention a aussi pour objet un comprimé de fénofibrate ou de l'un de ses dérivés, caractérisé en ce qu'il comprend :
- 23,09% en poids de fénofibrate ou de l'un de ses dérivés micronisé ;
- 0,82% de laurylsulfate de sodium micronisé ;
- 0,49% de silice colloïdale anhydre ;
- 29,4% en poids de cellulose microcristalline et 38,81% en poids de lactose monohydraté ;
- 4,9% en poids de croscarmellose sodique ;
- 0,5% en poids de stéarate de magnésium ;
- 2% en poids d'un agent de pelliculage ;
   les pourcentages en poids ci-dessus étant exprimés par rapport au poids total du comprimé.

Le comprimé ci-dessus est adapté pour une formulation pharmaceutique de fénofibrate ou de l'un de ses dérivés dosée à 160 mg par comprimé.

L'invention est également relative à un comprimé de fénofibrate ou de l'un de ses dérivés, caractérisé en ce qu'il comprend :
- 23,56% en poids de fénofibrate ou de l'un de ses dérivés micronisé ;
- 0,84% en poids de laurylsulfate de sodium ;
- 0,5% en poids de silice colloïdale anhydre ;
- 30% en poids de cellulose microcristalline et 39,6% en poids de lactose monohydratée ;
- 5 % en poids de croscarmellose sodique ; et
- 0,5 % en poids de stéarate de magnésium.

Les pourcentages en poids ci-dessus étant exprimés par rapport au poids total du comprimé.

Le comprimé tel que défini ci-dessus est adapté à la réalisation d'une formulation pharmaceutique de fénofibrate ou de l'un de ses dérivés dosée à 67 mg par comprimé.

L'invention concerne aussi un comprimé de fénofibrate ou de l'un de ses dérivés, caractérisé en ce qu'il comprend :
- 23,56% de fénofibrate ou de l'un de ses dérivés micronisé ;
- 0,84% en poids de laurylsulfate de sodium micronisé ;
- 0,5% en poids de silice colloïdale anhydre ;
- 30% en poids de cellulose microcristalline et 39,6% en poids de lactose monohydratée ;
- 5% en poids de croscarmellose sodique ; et
- 0,5% en poids de stéarate de magnésium.
   Les pourcentages en poids ci-dessus étant exprimés par rapport au poids total de la composition finale.

Le comprimé tel que défini ci-dessus est adapté à la réalisation d'une formulation pharmaceutique à base de fénofibrate ou de l'un de ses dérivés dosée à 200 mg par comprimé.

La présente invention est en outre illustrée, sans pour autant être limitée, par les figures et les exemples suivants.

### FIGURES

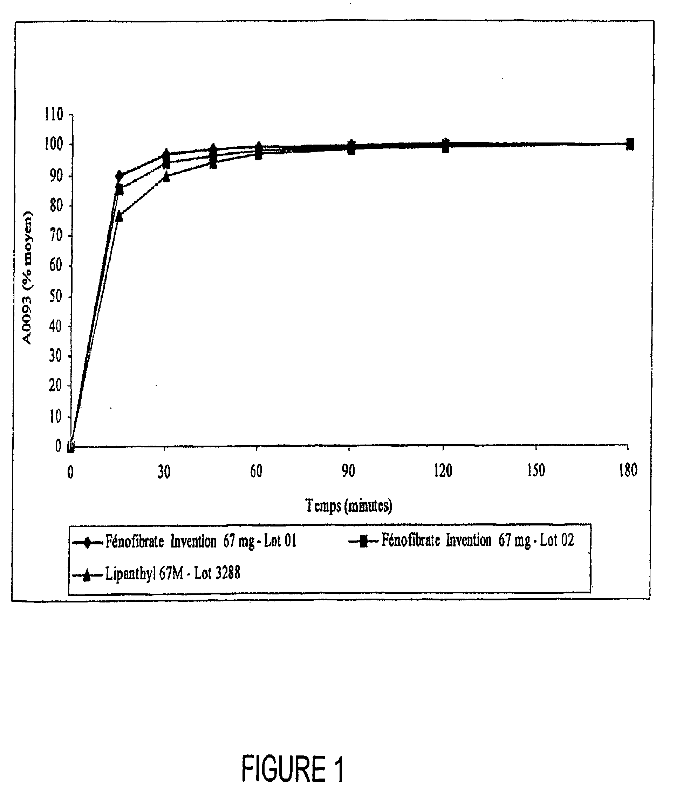

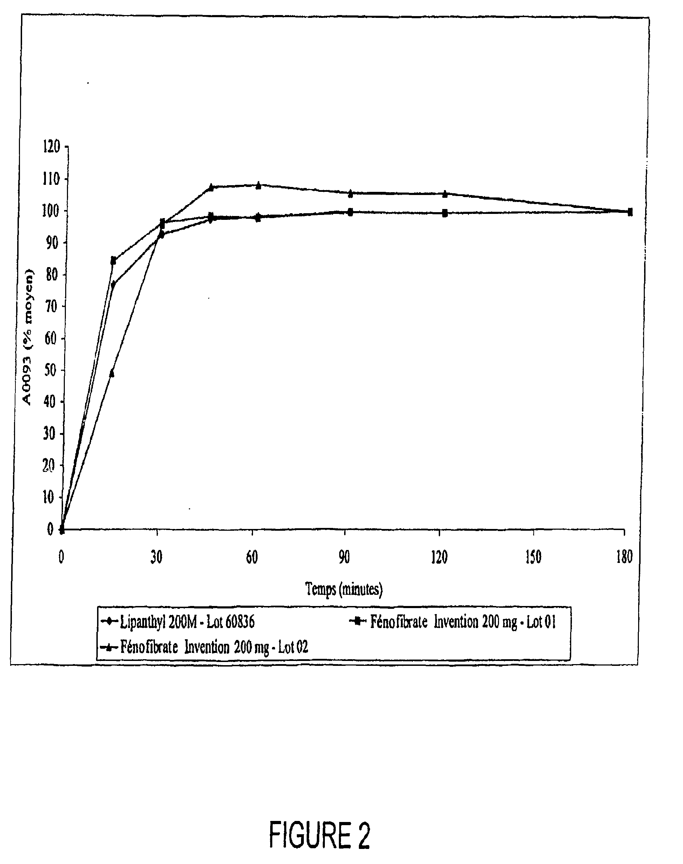

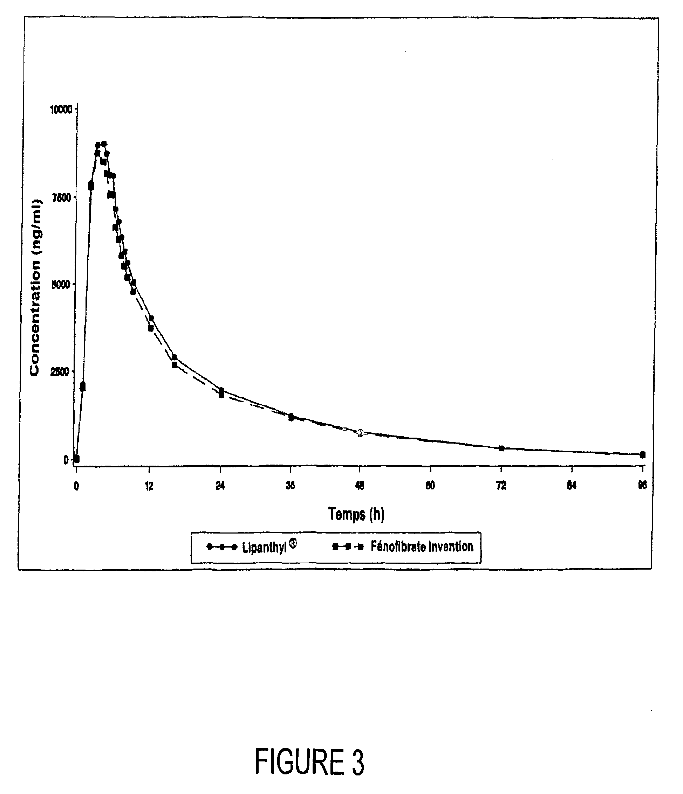

**La** **Figure 1** illustre une étude comparative du profil de dissolution *in vitro* les comprimés de fénofibrate à 67 mg selon l'invention et des gélules de fénofibrate à 67 mg commercialisées sous la marque Lipanthyl®.
**La** **Figure 2** illustre une étude comparative du profil de dissolution *in vitro* les comprimés de fénofibrate à 200 mg selon l'invention et des gélules de fénofibrate à 200 mg commercialisées sous la marque Lipanthyl®.
**La** **figure 3** illustre une étude comparative du profil de pharmacocinétique *in vivo* entre des comprimés de fénofibrate à 200 mg selon l'invention et des gélules de fénofibrate à 200 mg commercialisées sous la marque Lipanthyl®.

### EXEMPLES

### MATERIEL UTILISE DANS LES EXEMPLES

### 1 - DESCRIPTION DU MATERIEL ET DU PROCEDE DE FABRICATION

### Matériel

Tous les matériels sont mis à disposition selon les procédures en vigueur sur le site de fabrication, conformément aux Bonnes Pratiques de Fabrication :
- Mélangeur BÖHLE (ou équivalent)
- Microniseur ALPINE 200 AS (ou équivalent)
- Granulateur ALEXANDER WERK (ou équivalent)
- Calibreur FREWITT MG 333 (ou équivalent)
- Machine à comprimer rotative KILLIAN TX26 (ou équivalent)
- Poinçons de format 18x8R8 avec barre de confort
- Turbine de pelliculage WALTHER TROWAL (ou équivalent)
- Conditionneuse KLÖCKNER HÄNSEL (ou équivalent)

### Description du procédé de fabrication

Toutes les étapes intervenant au cours de la fabrication sont menées en conformité avec les Bonnes Pratiques de Fabrication.

### ETAPE 0 : PESEES

Effectuer les pesées.

### ETAPE 1 : MELANGE A

Introduire dans la cuve du mélangeur le fénofibrate et le laurylsulfate de sodium exactement pesées, si nécessaire préalablement tamisées sur tamis inox d'ouverture de maille 1,0 mm. Mélanger à environ 6 tr/min pendant environ 30 minutes.

### ETAPE 2 : MICRONISATION

Microniser le mélange A pendant 40 minutes. Les paramètres de micronisation sont :
- Pression d'air : 6 bars
- Débit d'air: 180 m³/h
- Vitesse : 25 kg/h

### ETAPE 3 : MELANGE B

Introduire dans la cuve du mélangeur le co-micronisat, le lactose monohydraté, la silice colloïdale anhydre, la cellulose microcristalline type 102 et le croscarmellose sodique si nécessaire préalablement tamisées sur tamis inox d'ouverture de maille 1,0 mm. Mélanger environ 20 minutes à 6 tr/min.

Ajouter la première fraction de stéarate de magnésium. Mélanger environ 5 minutes à 6 tr/min.

### Etape 4 : GRANULATION SECHE

Compacter le mélange B en utilisant les paramètres suivants :
- Force de compactage 55 KN
- Epaisseur des compactes 1,25 mm

### ETAPE 5 : CALIBRATION

Calibrer le grain obtenu à l'étape précédente avec une grille d'ouverture de maille 1,25 mm à l'aide du calibreur.

Introduire dans le mélangeur final le grain calibré ²pour homogénéisation. Mélanger pendant environ 5 minutes à 6 tr/min

### ETAPE 6 : MELANGE FINAL

Après avoir tamisé sur grille de diamètre 1,0 mm, introduire dans le mélangeur le solde de stéarate de magnésium exactement pesé. Mélanger à environ 6 tr/min pendant 3 minutes.

### ETAPE 7 : COMPRESSION

Equiper la machine à comprimer avec les poinçons adéquats. Procéder au réglage de la machine de manière à obtenir des comprimés conformes aux spécifications suivantes :

**Tableau 1**

| | Spécifications |
|---|---|
| Format des poinçons | 18x8R8 |
| Masse moyenne | 679,12 mg +/- 3 % |
| Uniformité de masse | Conforme Ph. Eur. (2.9.5) |
| Dureté sur 10 comprimés nus | 60 à 100N |
| Friabilité sur 20 comprimés ≤ nus | 1,00 % |
| Désagrégation sur 6 comprimés nus | ≤15 min |

### ETAPES 8 ET 9: PELLICULAGE

Dans un récipient de volume adéquate, introduire la quantité nécessaire d'eau purifiée pour la préparation d'une suspension de pelliculage dosée à 15 % (m/m). Ajouter l'OPADRY® OYL 28900 blanc exactement pesé à l'eau purifiée sous agitation. Laisser reposer la suspension au moins une heure sous agitation minimale.

La quantité de solution de pelliculage peut être ajustée en fonction de l'équipement utilisé pour le pelliculage. Introduire les comprimés nus dans la turbine et commencer le pelliculage :
- Température produit : 38 °C — 40 °C
- Débit d'air : 28 g/min
- Pression de pulvérisation : 2,5 bar
- Vitesse de la turbine : 25 rpm
   de manière à obtenir des comprimés conformes aux spécifications suivantes :

**Tableau 2**

| | Spécifications |
|---|---|
| Masse moyenne | 692,98 mg +/- 3% |
| Uniformité de masse | Conforme Ph. Eur. (2.9.5) |
| Dureté sur 10 comprimés nus | 70 à 110 N |
| Désagrégation sur 6 comprimés nus | ≤ 15 min |

### ETAPE 10 : CONDITIONNEMENT

Conditionner les comprimés en blister PVC/PVDC/Alu.

### 2 - CONTROLE DES ETAPES CRITIQUES ET INTERMEDIAIRES

### ETAPE 1 : MELANGE A

- Vérifier les pesées et les numéros de contrôles des matières premières.
- Vérifier la vitesse de rotation : environ 6 tr/min.
- Vérifier la durée du mélange : environ 30 minutes.
- Vérifier l'aspect du mélange.

### ETAPE 2 : MICRONISATION

- Vérifier les paramètres de micronisation
- Vérifier la distribution granulométrique du co-micronisat :
   75 % des particules ont une taille ³ 1,75 µm +/- 0,17 µm
   25 % des particules ont une taille ³ 4,7 µm +/- 0,47 µm
   90 % des particules ont une taille comprise entre 0,824 µm +/- 0,8 et 6,5 µm +/- 0,65 µm
   d(0,5) = 3,143 µm +/- 0,31 µm

### ETAPE 3 : MELANGE B

- Vérifier les pesées et les numéros de contrôles des matières premières.
- Vérifier la vitesse de rotation : environ 6 tr/min.
- Vérifier la durée du mélange : environ 20 minutes.
- Vérifier l'aspect du mélange.

Après ajout du stéarate de magnésium:
- Vérifier la vitesse de rotation : environ 6 tr/min.
- Vérifier la durée du mélange : environ 5 minutes.
- Vérifier l'aspect du mélange.

### ETAPE 4 : GRANULATION SECHE

- Vérifier les paramètres de compactage.
- Vérifier l'aspect du grain.

### ETAPE 5 : CALIBRATION

- Vérifier la conformité des grilles de calibrage (1,25 mm).
- Vérifier la vitesse de rotation lors de l'homogénéisation : environ 6 tr/min.
- Vérifier la durée de l'homogénéisation : environ 5 minutes.

### ETAPE 6 : MELANGE FINAL

- Vérifier la durée et la vitesse : environ 3 minutes à 6 tr/min.
- Vérifier l'aspect.
- Contrôler l'humidité résiduelle [3 g - 70 °C — 15 min] : ≤ 10,0 p. cent.

### ETAPE 7 : COMPRESSION

- Vérifier le format des poinçons :18x8R8 avec barre de confort
- Vérifier périodiquement les paramètres de compression.
- Enregistrer les réglages de la machine.

### ETAPES 8 ET 9: PELLICULAGE

- Vérifier la vitesse, la durée de préparation et l'aspect (lisse et peu de bulles d'air) de la solution de pelliculage.
- Vérifier et enregistrer les paramètres de l'équipement de pelliculage.
- Vérifier la conformité des comprimés pelliculés

### ETAPE 10 : CONDITIONNEMENT

- i Vérifier la conformité des matériaux de conditionnement
- i Vérifier l'étanchéité.
- i Vérifier la présence des informations réglementaires (numéro de lot, ≡ date de péremption).

### EXEMPLE 1: Préparation de comprimés dosés à 67 mg de fénofibrate.

### 1.1 composition qualitative et quantitative des comprimés

L La composition qualitative et quantitative des comprimés dosés à 67 mg de fénofibrate est présentée dans le Tableau 3, à la fin de la présente description.

### 1.2. Procédé de fabrication et analyse.

### 1.2. Procédé de fabrication et analyse

### Description du procédé de fabrication

Tc utes les étapes intervenant au cours de la fabrication sont menées en conformité avec les Bonnes Pratiques de Fabrication.

Vérifier la propreté du matériel et de la zone de travail.

### Etape 0 : PESEES

Effectuer les pesées.

### Etape 1 : MELANGE A

Introduire dans la cuve du mélangeur le fénofibrate et le laurilsulfate de sodium exactement pesées, si nécessaire préalablement tamisées sur tamis inox d'ouverture de maille 1,0 mm. Mélanger à environ 6 tr/min pendant environ 30 minutes.

### Etape 2 : MICRONISATION

Microniser le mélange A pendant 40 minutes. Les paramètres de micronisation sont :
- Pression d'air : 6 bars
- Débit d'air: 180 m³/h
- Vitesse : 25 kg/h

### Etape 3 : MELANGE B

Introduire dans la cuve du mélangeur le co-micronisat, le lactose monohydraté, la silice colloïdale anhydre, la cellulose microcristalline type 102 et le croscarmellose sodique si nécessaire préalablement tamisées sur tamis inox d'ouverture de maille 1,0 mm. Mélanger environ 20 minutes à 6 tr/min.

Ajouter la première fraction de stéarate de magnésium. Mélanger environ 5 minutes à 6 tr/min.

### Etape 4 : GRANULATION SECHE

Compacter le mélange B en utilisant les paramètres suivants :
- Force de compactage 55 KN
- Epaisseur des compactes 1,25 mm

### Etape 5 : CALIBRATION

Calibrer le grain obtenu à l'étape précédente avec une grille d'ouverture de maille 1,25 mm à l'aide du calibreur.

Introduire dans le mélangeur final le grain calibré pour homogénéisation. Mélanger pendant environ 5 minutes à 6 tr/min

### Etape 6 : MELANGE FINAL

Après avoir tamisé sur grille de diamètre 1,0 mm, introduire dans le mélangeur le solde de stéarate de magnésium exactement pesé. Mélanger à environ 6 tr/min pendant 3 minutes.

### Etape 7 : COMPRESSION

Equiper la machine à comprimer avec les poinçons adéquats. Procéder au réglage de la machine de manière à obtenir des comprimés conformes aux spécifications présentées dans le Tableau 4 ci-dessous.

**Tableau 4**

| | **Spécifications** |
|---|---|
| Format des poinçons | 10R10 |
| Masse moyenne | 284,37 mg ± 5% |
| Uniformité de masse | Conforme Ph. Eur. (2.9.5) |
| Dureté sur 10 comprimés nus | 60 N ± 20 N |
| Friabilité sur 20 comprimés nus | < 1,0 % |
| Désagrégation sur 6 comprimés nus | < 3 min |

### Etape 10 : CONDITIONNEMENT

Conditionner les comprimés en blister PVC/PVDC/Alu.

### Résultats sur Fénofibrate micronisé avec SLS

### Distribution granulométrique Coulter

Spécifications granulométriques de co-micronisation :
100 % <20 µm
50 % < 6 µm
20 % < 4µm

### Distribution granulométrique par tamis superposés du grain

La distribution granulométrique de la composition est représentée dans le Tableau 5 ci-après.

**Tableau 5**

| **Ouverture de maille (µm)** | **Refus (%)** | | | **Refus cumulés (%)** | | |
|---|---|---|---|---|---|---|
| | **22404** | **22449** | **22450** | **22404** | **22449** | **22450** |
| 710 | 11,79 | 22,74 | 17,90 | 11,79 | 22,74 | 17,90 |
| 500 | 3,64 | 6,59 | 5,26 | 15,43 | 29,33 | 23,15 |
| 355 | 3,49 | 4,40 | 3,64 | 18,92 | 33,73 | 26,79 |
| 250 | 4,80 | 4,91 | 5,97 | 23,72 | 38,64 | 32,76 |
| 180 | 23,21 | 9,48 | 12,44 | 46,93 | 48,12 | 45,20 |
| 125 | 17,08 | 18,80 | 22,85 | 64,01 | 66,92 | 68,05 |
| 90 | 19,22 | 18,94 | 16,99 | 83,23 | 85,86 | 85,04 |
| Fond | 16,77 | 14,14 | 14,96 | 100,00 | 100,00 | 100,00 |

Caractéristiques pharmacotechniques - Mélange final avant compactage

Les caractéristiques pharmacotechniques de la composition sont représentées dans le Tableau 6 ci-dessous.

**Tableau 6**

| **ESSAIS** | | **Lot 01** | **Lot 02** |
|---|---|---|---|
| Ecoulement (s) (100 g) | | Infini | Infini |
| Volume apparent (ml) (100g) | - V0 | 242 | 234 |
| | - V10 | 234 | 230 |
| | -V500 | 178 | 175 |
| | -V1250 | 178 | 169 |
| | - V2500 | - | 169 |
| Aptitude au tassement (ml) | - V10-V500 | 56 | 55 |
| Masse volumique app. (g/ml) | - m/V0 | 0,41 | 0,43 |
| | - mN1250 | 0,56 | - |
| | - mN2500 | - | 0,59 |
| Humidité résiduelle (%) (75 % - 10 min) | | 2,89 | 2,43 |

### Distribution granulométrique sur tamis superposés

La distribution granulométrique de la composition est données dans le Tableaux 7 ci-dessous.

**Tableau 7**

| **Ouverture de maille (µm)** | **Refus cumulés en %** | |
|---|---|---|
| | **Lot 01** | **Lot 02** |
| 710 | 1,99 | 1,29 |
| 500 | 4,77 | 3,87 |
| 355 | 8,45 | 7,63 |
| 250 | 12,13 | 16,65 |
| 180 | 17,00 | 23,42 |
| 125 | 54,08 | 61,12 |
| 90 | 76,24 | 84,96 |
| Fond | 100,00 | 100,00 |

### Caractéristiques pharmacotechniques - Mélange final avant compression

Les caractéristiques pharmacotechniques du mélange final avant compression sont représentées dans le Tableau 8 ci-dessous.

**Tableau 8**

| **ESSAIS** | | **Lot 01** | **Lot 02** |
|---|---|---|---|
| Ecoulement (s) * (100 g) | | 5,52 | 4,84 |
| Volume apparent (ml) (100 g) | - V0 | 160 | 150 |
| | - V10 | 152 | 140 |
| | -V500 | 134 | 127 |
| | - V1250 | 130 | 124 |
| | - V2500 | 129 | 123 |
| Aptitude au tassement (ml) | - V10-V500 | 18 | 13 |
| Masse volumique app. (g/ml) | - m/V0 | 0,62 | 0,66 |
| | - m/V1250 | 0,77 | 0,80 |
| | - m/V2500 | 0,78 | 0,81 |
| Humidité résiduelle (%) (75%-10 | min) | 2,80 | 2,24 |

| | | | |
|---|---|---|---|
| * : Le temps d'écoulement réalisé dans un entonnoir en verre (Cf. Ph.Eur. en vigueur) laisse apparaître des phénomènes électrostatiques au niveau de la poudre qui disparaissent au niveau industriel alimentation INOX). Ce phénomène ne reflète pas une réalité opérationnelle. | | | |

Les caractéristiques de granulométrie du mélange final avant compression sont représentées dans le Tableau 9 ci-dessous.

**Tableau 9**

| **Ouverture de maille (µm)** | **Refus cumulés en %** | |
|---|---|---|
| | **Lot 01** | **Lot 02** |
| 710 | 22,61 | 32,77 |
| 500 | 32,07 | 42,93 |
| 355 | 37,75 | 48,80 |
| 250 | 43,53 | 53,78 |
| 180 | 48,90 | 58,07 |
| 125 | 59,86 | 66,63 |
| 90 | 68,82 | 74,10 |
| Fond | 100,00 | 100,00 |

### Résultats des essais pharmacotechniques.

Les résultats des essais pharmacotechniques sont représentés dans le Tableau 10, à la fin de la présente description.

### Dissolution in vitro comparée

On a comparé les profils de libération du fénofibrate *in vitro* entre les comprimés dosés à 67 mg de fénofibrate, des lots n°01 et n° 02, et préparés conformément au procédé selon l'invention, avec les gélules au même dosage commercialisées sous la marque Lipanthyl®.

Les résultats sont représentés sur la Figure 1.

Les comprimés des lots n° 01 et n° 02, préparés conformément au procédé de l'invention, possèdent un profil de dissolution *in vitro* du fénofibrate très similaire, sinon identique, à celui des gélules de Lipanthyl® 67 mg.

### Exemple 2 : Préparation de comprimés dosés à 200 mg de fénofibrate.

### 2.1 composition qualitative et quantitative des comprimés

### Tableau 11 : composition qualitative et quantitative des comprimés dosés à 200 mg.

La composition qualitative et quantitative des comprimés est représentée dans le Tableau 11 ci-dessous.

**Tableau 11**

| **NOM DES COMPOSANTS** | **FORMULE** | | **FONCTION** | **REFERENCE AUX NORMES** |
|---|---|---|---|---|
| | **Centésimale** | **Unitaire** | | |
| ***Principe actif*** | | | | |
| - Fénofibrate (micronisé) | 23,56 | 200,00 | Actif | Ph.Eur.3^{ème} édition Monographie 1322 en vigueur |
| **Autres composants** | 0,84 | 7,14 | Mouillant | |
| - Sodium Laurylsulfate | 39,60 | 336,16 | Diluant | Ph.Eur.3^{ème} édition Monographie 0098 en vigueur |
| - Lactose monohydraté | 0,50 | 4,24 | Agent d'écoulement | Ph.Eur.3^{ème} édition Monographie 0187 en vigueur |
| | 5,00 | 42,44 | Désintégrant | |
| - Silice colloïdale anhydre | 30,00 | 254,67 | Diluant/Liant | |
| - Croscarmellose sodique | 0,50 | 4,24 | Lubrifiant | Ph.Eur.3ème édition Monographie 0434 en vigueur |
| - Cellulose microcristalline | | | | Ph.Eur.3^{éme} édition Monographie 0985 en vigueur |
| - Stéarate de magnésium | | | | Ph.Eur.3^{ème} édition Monographie 0316 en vigueur |
| | | | | Ph.Eur.3^{ème} édition Monographie 0229 en vigueur |
| Masse unitaire (mg/comprimé) | | 848,90 | | |

### 2.2. Procédé de fabrication et analyse

### Description du procédé de fabrication

Toutes les étapes intervenant au cours de la fabrication sont menées en conformité avec les Bonnes Pratiques de Fabrication.

Vérifier la propreté du matériel et de la zone de travail.

### Etape 0 : PESEES

Effectuer les pesées.

### Etape 1 : MELANGE A

Introduire dans la cuve du mélangeur le fénofibrate et le laurylsulfate de sodium exactement pesées, si nécessaire préalablement tamisées sur tamis inox d'ouverture de maille 1,0 mm. Mélanger à environ 6 tr/min pendant environ 30 minutes.

### Etape 2 : MICRONISATION

Microniser le mélange A pendant 40 minutes. Les paramètres de micronisation sont :
- Pression d'air : 6 bars
- Débit d'air: 180 m³/h
- Vitesse : 25 kg/h

### Etape 3 : MELANGE B

Introduire dans la cuve du mélangeur le co-micronisat, le lactose monohydraté, la silice colloïdale anhydre, la cellulose microcristalline type 102 et le croscarmellose sodique si nécessaire préalablement tamisées sur tamis inox d'ouverture de maille 1,0 mm. Mélanger environ 20 minutes à 6 tr/min.

Ajouter la première fraction de stéarate de magnésium. Mélanger environ 5 minutes à 6 tr/min.

### Etape 4 : GRANULATION SECHE

Compacter le mélange B en utilisant les paramètres suivants :
- Force de compactage 55 KN
- Epaisseur des compactes 1,25 mm

### Etape 5 : CALIBRATION

Calibrer le grain obtenu à l'étape précédente avec une grille d'ouverture de maille 1,25 mm à l'aide du calibreur.

Introduire dans le mélangeur final le grain calibré pour homogénéisation. Mélanger pendant environ 5 minutes à 6 tr/min

### Etape 6 : MELANGE FINAL

Après avoir tamisé sur grille de diamètre 1,0 mm, introduire dans le mélangeur le solde de stéarate de magnésium exactement pesé. Mélanger à environ 6 tr/min pendant 3 minutes.

### Etape 7 : COMPRESSION

Equiper la machine à comprimer avec les poinçons adéquats. Procéder au réglage de la machine de manière à obtenir des comprimés conformes aux spécifications représentées dans le Tableau 12 ci-dessous.

**Tableau 12**

| | **Spécifications** |
|---|---|
| Format des poinçons | 14R15 |
| Masse moyenne | 848,90 mg ± 5 % |
| Uniformité de masse | Conforme Ph. Eur. (2.9.5) |
| Dureté sur 10 comprimés nus | 70 N ± 30 N |
| Friabilité sur 20 comprimés nus | < 0,5 % |
| Désagrégation sur 6 comprimés nus | < 3 min |

### Etape 9 : CONDITIONNEMENT

Conditionner les comprimés en blister PVC/PVDC/Alu.

### Résultats sur Fénofibrate

### Caractéristiques pharmacotechniques du grain

Les caractéristiques pharmacotechniques du grain sont représentées dans le Tableau 13 ci-dessous.

**Tableau 13**

| **ESSAIS** | | | **Lot 22404** | **Lot 22449** | **Lot 22450** |
|---|---|---|---|---|---|
| Ecoulement (100 g) | | | Infini | Infini | Infini |
| Volume apparent (100 g) : | | | | | |
| | | V0 | 164 ml | 149 ml | 156 ml |
| | | V10 | 158 ml | 148 ml | 152 ml |
| | | V500 | 138 ml | 129 ml | 134 ml |
| | | V1250 | 137 ml | 128 ml | 132 ml |
| Aptitude au tassementV10-V500 | | | 20 ml | 19 ml | 18 ml |
| Masse volumique apparente | | | | | |
| (g/ml) | | | 0,614 | 0,669 | 0,640 |
| | m/V0 | | 0,736 | 0,779 | 0,757 |
| | m/V1250 | | 2,39 | 2,25 | 1,87 |
| Humidité résiduelle (%) | | | | | |
| (3 g - 15 *min -* 70°C) | | | | | |

### Distribution granulométrique sur tamis superposés du grain

La distribution granulométrique de la composition est représentée dans le Tableau 14 ci-dessous.

**Tableau 14**

| **Ouverture de maille** (µm) | **Refus (%)** | | | **Refus cumulés (%)** | | |
|---|---|---|---|---|---|---|
| | **22404** | **22449** | **22450** | **22404** | **22449** | **22450** |
| 710 | 11,79 | 22,74 | 17,90 | 11,79 | 22,74 | 17,90 |
| 500 | 3,64 | 6,59 | 5,26 | 15,43 | 29,33 | 23,15 |
| 355 | 3,49 | 4,40 | 3,64 | 18,92 | 33,73 | 26,79 |
| 250 | 4,80 | 4,91 | 5,97 | 23,72 | 38,64 | 32,76 |
| 180 | 23,21 | 9,48 | 12,44 | 46,93 | 48,12 | 45,20 |
| 125 | 17,08 | 18,80 | 22,85 | 64,01 | 66,92 | 68,05 |
| 90 | 19,22 | 18,94 | 16,99 | 83,23 | 85,86 | 85,04 |
| Fond | 16,77 | 14,14 | 14,96 | 100,00 | 100,00 | 100,00 |

### Caractéristiques pharmacotechniques - Mélange final avant compactage

Les caractéristiques pharmacotechniques du mélange final sont représentées dans le Tableau 15 ci-dessous.

**Tableau 15**

| **ESSAIS** | | **Lot 01** | **Lot 02** |
|---|---|---|---|
| Ecoulement (s) (100 g) | | Infini | Infini |
| Volume apparent (ml) (100 g) | - V0 | 242 | 234 |
| | - V10 | 234 | 230 |
| | - V500 | 178 | 175 |
| | -V1250 | 178 | 169 |
| | - V2500 | - | 169 |
| Aptitude au tassement (ml) | - V10-V500 | 56 | 55 |
| Masse volumique apparente (g/ml) | - m/V0 | 0,41 | 0,43 |
| | - m/V1250 | 0,56 | - |
| | - m2500 | - | 0,59 |
| Humidité résiduelle (%) (75%-10 min) | | 2,89 | 2,43 |

### Distribution granulométrique sur tamis superposés

La distribution granulométrique de la composition est représentée dans le Tableau 16 ci-dessous.

**Tableau 16**

| **Ouverture de maille (µm)** | **Refus cumulés en %** | |
|---|---|---|
| | **Lot 01** | **Lot 02** |
| 710 | 1,99 | 1,29 |
| 500 | 4,77 | 3,87 |
| 355 | 8,45 | 7,63 |
| 250 | 12,13 | 16,65 |
| 180 | 17,00 | 23,42 |
| 125 | 54,08 | 61,12 |
| 90 | 76,24 | 84,96 |
| Fond | 100,00 | 100,00 |

### Caractéristiques pharmacotechniques - Mélange final avant compression

Les caractéristiques pharmacotechniques du mélange final avant compression dans le Tableau 17 ci-dessous.

**Tableau 17**

| **ESSAIS** | | **Lot 01** | **Lot 02** |
|---|---|---|---|
| Ecoulement (s) * (100 g) | | 5,52 | 4,84 |
| Volume apparent (ml) (100g) | - V0 | 160 | 150 |
| | - V10 | 152 | 140 |
| | - V500 | 134 | 127 |
| | -V1250 | 130 | 124 |
| | - V2500 | 129 | 123 |
| Aptitude au tassement (ml) | - V10-V500 | 18 | 13 |
| Masse volumique app. (g/ml) | m/V0 | 0,62 | 0,66 |
| | - m/V1250 | 0,77 | 0,80 |
| | - mN2500 | 0,78 | 0,81 |
| Humidité résiduelle (%) (75 % - 10 min) | | 2,80 | 2,24 |

| | | | |
|---|---|---|---|
| * : Le temps d'écoulement réalisé dans un entonnoir en verre (Cf. Ph.Eur.en vigueur) laisse apparaître des phénomènes électrostatiques au niveau de la poudre qui disparaissent au niveau industriel (alimentation INOX). | | | |

Ce phénomène ne reflète pas une réalité opérationnelle.

### Distribution granulométrique sur tamis superposés

La distribution granulométrique est représentée dans le Tableau 18 ci-dessous.

**Tableau 18**

| **Ouverture de maille (µm)** | **Refus cumulés en %** | |
|---|---|---|
| | **Lot 01** | **Lot 02** |
| 710 | 22,61 | 32,77 |
| 500 | 32,07 | 42,93 |
| 355 | 37,75 | 48,80 |
| 250 | 43,53 | 53,78 |
| 180 | 48,90 | 58,07 |
| 125 | 59,86 | 66,63 |
| 90 | 68,82 | 74,10 |
| Fond | 100,00 | 100,00 |

Les résultats des contrôles des comprimés de fénofibrate sont représentés dans le Tableau 19, à la fin de la présente description.

### Dissolution in vitro comparée

On a comparé les profils de libération du fénofibrate *in vitro* entre les comprimés dosés à 200 mg de fénofibrate, des lots n°01 et n° 02, et préparés conformément au procédé selon l'invention, avec les gélules au même dosage commercialisées sous la marque Lipanthyl®.

Les résultats sont représentés sur la Figure 2. Les comprimés des lots n° 01 et n° 02, préparés conformément au procédé de l'invention, possèdent un profil de dissolution *in vitro* du fénofibrate très similaire, sinon identique, à celui des gélules de Lipanthyl® 200 mg.

### EXEMPLE 3 : Analyse comparative de pharmacocinétique in vivo entre des comprimés de fénofibrate dosés à 200 mg selon l'invention et des gélules de fénofibrate commercialisés sous la marque Lipanthyl® 200M, aux mêmes dosages.

Un groupe de 23 individus, respectivement 17 hommes et 6 femmes, âgés de 18 à 40 ans ont été sélectionnés pour l'étude et ont été divisés en deux sous-groupes, respectivement un premier groupe traité avec des comprimés de fénofibrate dosés à 200 mg selon l'invention et un second sous-groupe traité avec des gélules de fénofibrate dosés à 200 mg et commercialisés sous la marque Lipanthyl®.

Après le petit déjeuner, chaque individu a pris par la voie orale un comprimé de 200 mg selon l'invention ou une gélule à 200 mg Lipanthyl® avec 240 ml d'eau.

Puis, des échantillons d'un volume de 10 ml de sang veineux ont été recueillis sur chaque individu dans des tubes de verre héparinés, respectivement avant la prise orale du comprimé ou de la gélule de fénofibrate et aux temps 1, 2, 3, 4, 4.5, 5, 5.5, 6, 7, 8, 10, 12, 16 , 24, 48, 72 et 96 heures après la prise orale du comprimé ou de la gélule de fénofibrate.

La concentration d'acide fénofibrique, exprimée en ng/ml, a été mesurée dans chacun des échantillons de sang prélevé.

La moyenne de la concentration d'acide fénofibrique pour l'ensemble des individus de chacun des deux groupes a été calculée. Les résultats sont représentés sur la figure 3.

Les résultats de la figure 3 montrent que le profil de pharmacocinétique *in vivo* du fénofibrate, pour les individus ayant été traités avec des comprimés de fénofibrate à 200 mg selon l'invention est identique au profil pharmacocinétique du fénofibrate pour les individus traités avec les gélules de Lipanthyl® dosés à 200 mg.

### EXEMPLE 4 :

### Analyse comparative de pharmacocinétique in vivo entre des comprimés de fénofibrate dosés à 200 mg selon l'invention et des gélules de fénofibrate commercialisés sous la marque Lipanthyl® , au même dosage.

Un groupe de 26 individus, respectivement 12 hommes et 14 femmes âgés de 18 à 55 ans, ont été sélectionnés pour une étude comprenant deux phases de traitement de 96 heures chacune, à savoir une phase de traitement avec des comprimés de fénofibrate dosés à 200 mg selon l'invention et une phase de traitement avec des gélules de fénofibrate dosées à 200 mg et commercialisées sous la dénomination de spécialités pharmaceutiques Lipanthyl® 200 mg. Les deux phases de traitement sont séparées par une période de 14 jours tenant compte de la demi-vie de l'acide fénofibrique. La séquence de traitement de chaque patient a été randomisée.

Chaque patient a consommé un petit-déjeuner gras et énergétique, et a pris par voie orale cinq minutes après la fin du petit-déjeuner, un comprimé de 200 mg de fénofibrate selon l'invention et une gélule de 200 mg de Lipanthyl® avec 180 ml d'eau.

Des échantillons d'un volume de 10 ml de sang veineux ont été recueillis sur chaque individu dans des tubes de verre héparinés, respectivement avant la prise orale du comprimé ou de la gélule de fénofibrate et au temps 1, 2 , 3, 4, 4,5, 5, 5,5, 6, 6,5, 7, 7,5, 8, 9, 10, 12, 16 et 24 heures après la prise orale du comprimé ou de la gélule de fénofibrate.

La concentration d'acide fénofibrique, exprimée en ng/ml a été mesurée dans chacun des échantillons de sang prélevé.

La moyenne arithmétique de la concentration d'acide fénofibrique au cours du temps, pour l'ensemble des individus a également été calculée, après la phase de traitement par des comprimés de fénofibrate dosés à 200 mg selon l'invention et après la phase de traitement par des gélules de 200 mg de Lipanthyl®.

La courbe d'évolution de la moyenne arithmétique de la concentration plasmatique en acide fénofibrique en fonction du temps a été tracée.

La concentration plasmatique maximum (Cₘₐₓ), le temps nécessaire à l'obtention de la concentration plasmatique maximum (Tₘₐₓ) et l'aire sous la courbe de concentration plasmatique (ASC) ont été mesurés pour chaque traitement.

Les valeurs de ces variables, leur moyenne et leur intervalle ont été calculées pour chaque phase de traitement et regroupées dans le tableau 20 ci-dessous.

**TABLEAU 20**

| **Paramètre** | | **Comprimé selon l'invention 200mg** | **Gélule 200mg Arrow® Génériques** | **Gélule de Lipanthyl ®200mg** | **Comprimé de lipanthyl 160 mg** |
|---|---|---|---|---|---|
| **Cₘₐₓ** | moyenne | 10670 | 10384 | 13084 | 10592 |
| | Ecart-type | 1988 | 2148 | 2087 | - |
| **Tₘₐₓ** | moyenne | 5,25 | 5,50 | 5,50 | - |
| | Ecart-type | - | - | - | - |
| **ASC₀₋ₜ** | moyenne | 219718 | 205781 | 237068 | - |
| | Ecart-type | 69070 | 65951 | 65131 | - |
| **ASC**_{**0**-∞} | moyenne | 230144 | 213902 | 247157 | 167702 |
| | Ecart-type | 79070 | 73066 | 77619 | - |

| | | | | | |
|---|---|---|---|---|---|
| **ASC**₀₋ₜ : aire sous la courbe de concentration plasmatique mesurée entre 0 et 24 heures. **ASC**_{**0**-∞} : aire sous la courbe de concentration plasmatique calculée par extrapolation jusqu'à l'infini. | | | | | |

Les variables du tableau 20 ont été comparées dans le tableau 21 ci-dessous.

**Tableau 21**

| Paramètre | IC 90* -Comprimé selon l'invention | IC 90 -Gélule de Lipanthyl® 200mg | Résultat |
|---|---|---|---|
| **Cmax** | 74,6-88,2 | 72,5-85,6 | NS* |
| **Tₘₐₓ** | NS | NS | NS |
| **ASC**₀₋ₜ | 86,3-97,1 | 80,7-90,8 | NS |
| **ASC**_{0-∞} | 86,7-97,7 | 80,6-90,8 | NS |

| | | | |
|---|---|---|---|
| *IC 90 : Intervalle de confiance à 90% *NS : valeurs non statistiquement différentes | | | |

Les résultats combinés du tableau 21 ci-dessus montrent que le profil pharmacocinétique *in vivo* du fénofibrate pour les individus ayant subi la phase de traitement avec les comprimés de fénofibrate à 200 mg selon l'invention est identique au profil pharmacocinétique des individus ayant subi la phase de traitement par les gélules de Lipanthyl® dosées à 200 mg.

**Tableau 3 : composition qualitative et quantitative des comprimés à 67 mg**

| **NOM DES COMPOSANTS** | **FORMULE** | | **FONCTION** | **REFERENCE AUX NORMES** |
|---|---|---|---|---|
| | **Centésimale** | **Unitaire** | | |
| ***Principe actif*** | | | | |
| - Fénofibrate (micronisé) | 23,56 | 67,00 | Actif | Ph.Eur. Monographie 1322 |
| **Autres composants** | | | | |
| - Sodium Laurylsulfate | 0,84 | 2,39 | Mouillant | Ph.Eur Monographie 0098 |
| - Lactose monohydraté | 39,60 | 112,61 | Diluant | Ph.Eur. Monographie 0187 |
| - Silice colloïdale anhydre | 0,50 | 1,42 | Agent d'écoulement | Ph.Eur. Monographie 0434 |
| Croscarmellose sodique | 5,00 | 14,22 | Désintégrant | Ph.Eur. Monographie 0985 |
| - Cellulose microcristalline | 30,00 | 85,31 | Diluant/Liant | Ph.Eur. Monographie 0316 |
| - Stéarate de magnésium | 0,50 | 1,42 | Lubrifiant | Ph.Eur. Monographie 0229 |
| Masse unitaire (mg/comprimé) | | 284,38 | | |

**Tableau 10 : Résultats des essais pharmacotechniques pour les comprimés de fénofibrate dosés à 67mg**

| **Contrôles** | | | **Normes** | **Résultats** | | | | | |
|---|---|---|---|---|---|---|---|---|---|
| | | | | **lot 01** | | | **lot 02** | | |
| | | | | **Début** | **Milieu** | **Fin** | **Début** | **Milieu** | **Fin** |
| ***Caractères*** | | | | | | | | | |
| - Apparence | | | comp. rond | conforme | conforme | conforme | conforme | conforme | conforme |
| - Couleur | | | blanc | conforme | conforme | conforme | conforme | conforme | conforme |
| - Odeur | | | inodore | conforme | conforme | conforme | conforme | conforme | conforme |
| ***Essais*** | | | | | | | | | |
| - Uniformité de masse | | | | | | | | | |
| | • comprimé | | conforme | conforme | conforme | conforme | conforme | conforme | conforme |
| | CV (%) | | | 2,60 | 2,43 | 2,28 | 2,43 | 2,13 | 1,90 |
| - Masse moyenne (mg) | | | | | | | | | |
| | • comprimé | | 270- 298,6 | 285,05 | 286,23 | 280,30 | 286,36 | 286,40 | 284,35 |
| - Désagrégation (min) | | | < 5 min | 1 min 37 | 1 min 06 | 1 min 05 | 41 s | 47 s | 45 s |
| - Dureté | | (N) | 60±20 N | 77,60 | 55,20 | 46,80 | 51,40 | 45,60 | 49,50 |
| - Uniformité de teneur | | | | conforme | conforme | conforme | conforme | conforme | conforme |
| | | - moyenne | | 61,78 | 65,40 | 66,47 | 66,99 | 65,36 | 69,15 |
| | | -CV% | CV<10% | 4,83 | 2,35 | 2,82 | 6,22 | 2,66 | 3,31 |
| - Dissolution in vitro à 30 min | | | | | | | | | |
| Libération dans SLS 2 % | | | | conforme | conforme | conforme | conforme | conforme | conforme |
| ***Identification et dosage*** | | | > 75 % | | | | | | |
| - Identification du Fénofibrate | | | | | | | | | |
| | | | tr témoin = tr | | | | | | |
| | | | essai | conforme | conforme | conforme | conforme | conforme | conforme |
| - Dosage du Fénofibrate | | | 63,65 à 70,35 | | 64,59 | | | 66,90 | |
| (mg/cpé) | | | | | | | | | |
| ***Essais complémentaires*** | | | | | | | | | |
| - Humidité résiduelle (%) | | | < 5 | 2,67 | 2,37 | 2,34 | 2,75 | 2,55 | 2,47 |
| - Friabilité (%) | | | < 0,5 | 0,16 | 0,16 | 0,095 | 0,34 | 0,26 | 0,43 |

**Tableau 19 : Résultats des contrôles des comprimés de fénofibrate selon l'invention dosés à 200 mg**

| **Contrôles** | | | **Normes** | **Résultats** | | | | | |
|---|---|---|---|---|---|---|---|---|---|
| | | | | **Lot 01** | | | **Lot 02** | | |
| | | | | **Début** | **Milieu** | **Fin** | **Début** | **Milieu** | **Fin** |
| ***Caractères*** | | | | | | | | | |
| - Apparence | | | comp rond | conforme | conforme | conforme | conforme | conforme | conforme |
| - Couleur | | | blanc | conforme | conforme | conforme | conforme | conforme | conforme |
| - Odeur | | | inodore | conforme | conforme | conforme | conforme | conforme | conforme |
| ***Essais*** | | | | | | | | | |
| - Uniformité de masse | | | | | | | | | |
| | • comprimé | | conforme | conforme | conforme | conforme | conforme | conforme | conforme |
| | CV (%) | | | 1,17 | 0,73 | 1,25 | 0,61 | 0,73 | 0,76 |
| - Masse moyenne (mg) | | | | | | | | | |
| | • comprimé | | 806-891 | 843,48 | 854,89 | 861,42 | 848,18 | 849,63 | 849,41 |
| - Désagrégation (min) | | | < 3 min | 1 min 16 | 1 min 20 | 1 min 20 | 1 min 06 | 1 min 09 | 1 min 22 |
| - Dureté (N) | | | 70±30N | 48,70 | 60,90 | 63,20 | 79,20 | 86,10 | 83,00 |
| - Uniformité de teneur | | | | conforme | conforme | conforme | conforme | conforme | conforme |
| | | - Moyenne | | 201,79 | 192,55 | 187,79 | 198,07 | 188,59 | 191,95 |
| | | -CV% | CV < 10% | 5,09 | 1,80 | 6,79 | 5,81 | 6,00 | 6,78 |
| - Dissolution in vitro à 30 min* | | | > 75 % | conforme | conforme | conforme | conforme | conforme | conforme |
| Libération dans SLS 2% | | | | | | | | | |
| ***Identification et dosage*** | | | | | | | | | |
| - Identification du Fénofibrate | | | tr témoin = | conforme | conforme | conforme | conforme | conforme | conforme |
| | | | tr essai | | | | | | |
| -Dosage du Fénofibrate (mg/cpé) | | | 190,0 à 210,0 | | 193,9 | | | 199,1 | |
| ***Essais complémentaires :*** | | | | | | | | | |
| - Humidité résiduelle (%) | | | < 5 | 2,97 | 3,07 | 2,70 | 2,54 | 2,50 | 2,58 |
| - Friabilité (%) | | | < 0,5 | 0,12 | 0,23 | 0,11 | 0,34 | 0,28 | 0,24 |

## Revendications

1. Procédé pour la fabrication d'une composition pharmaceutique contenant le principe actif fénofibrate ou de l'un de ses dérivés, éventuellement sous la forme d'une association du fénofibrate ou de son dérivé avec un second principe actif, sous la forme de comprimés, **caractérisé en ce qu'**il comprend les étapes suivants :
(a) préparer un mélange de fénofibrate ou de l'un de ses dérivés, ou de l'association avec un second principe actif, et d'au moins un agent tensioactif solide, dans une proportion de (i) 91 % à 99 % en poids de fénofibrate ou de son dérivé et (ii) 1 % à 9 % en poids dudit ou desdits agent(s) tensioactif(s) solide(s);
(b) microniser le mélange de fénofibrate ou de l'un de ses dérivés, éventuellement sous la forme d'une association avec un second principe actif, et d'agent(s) tensioactif(s) obtenu à l'étape (a), afin d'obtenir un micronisat du fénofibrate, ou de l'association avec le second principe actif, et du ou des agent(s) tensioactif(s) ;
(c) additionner au moins un agent anti-statique au co-micronisat préparé à l'étape (b);
(d) additionner au mélange obtenu à l'étape (c) au moins un agent diluant, au moins un agent désintégrant et au moins un agent lubrifiant, afin d'obtenir un mélange solide correspondant à la phase interne du comprimé ;
(e) compacter, selon une étape de granulation par voie sèche, le mélange solide obtenu à l'étape (d) afin d'obtenir la phase interne finale du comprimé;
(f) mélanger la phase interne du comprimé préparée à l'étape (e) avec une phase externe comprenant au moins un agent lubrifiant, puis effectuer une compression de la composition afin d'obtenir la composition pharmaceutique contenant le fénofibrate ou de l'un de ses dérivés sous la forme de comprimés.

2. Procédé selon la revendication 1, **caractérisé en ce qu'**à l'étape (a), le mélange de fénofibrate ou de l'un de ses dérivés, éventuellement sous la forme d'une association avec un second principe actif, et d'agent(s) tensioactif(s) comprend une proportion de (i) 95 % à 98 % en poids de fénofibrate ou de l'association et (ii) 2 % à 5 % en poids d'agent(s) tensioactif(s).

3. Procédé selon l'une des revendications 1 et 2, **caractérisé en ce qu'**à l'étape (b), le co-micronisat comprend des particules possédant une taille comprise entre 0,1 et 20 µm.

4. Procédé selon l'une des revendications 1 à 3, **caractérisé en ce qu'**à l'étape (c), le ou les agent(s) antistatique(s) est (sont) ajouté(s) à raison de 0,1 % à 5 % en poids, de préférence de 0,2 % à 2 % en poids, par rapport au poids total de la composition.

5. Procédé selon l'une des revendications 1 à 4, **caractérisé en ce qu'**à l'étape (d), le ou les agent(s) diluant(s) est (sont) ajouté(s) à raison de 40 % à 80 % en poids, de préférence entre 50 % et 75 % en poids, par rapport au poids total de la composition.

6. Procédé selon l'une des revendications 1 à 5, **caractérisé en ce qu'**à l'étape (d), le ou les agent(s) désintégrant(s) est (sont) ajouté(s) à raison de 1 % à 20 % en poids, de préférence de 3 à 6 % en poids, par rapport au poids total de la composition.

7. Procédé selon l'une des revendications 1 à 6, **caractérisé en ce qu'**à l'étape (d), le ou les agent(s) lubrifant(s) est (sont) ajouté(s) à raison de 0,1 % à 2 % en poids, de préférence de 0,2 % à 1 % en poids, par rapport au poids total de la composition.

8. Procédé selon l'une quelconque des revendications 1 à 7, **caractérisé en ce que** le fénofibrate ou de son dérivé est présent à raison de 20 % à 50 % en poids, par rapport au poids total de la composition.

9. Procédé selon la revendication 8, **caractérisé en ce que** le fénofibrate ou son dérivé est en association avec un second principe actif.

10. Procédé selon la revendication 9, **caractérisé en ce que** le second principe actif est choisi parmi la metformine, la cobalamine, l'acide folique, la bétaïne, la N-acétylcystéine, la vitamine E et un inhibiteur de HGMCoA.

11. Procédé selon l'une des revendications 1 à 10, **caractérisé en ce que** le ou les agent(s) tensioactif(s) est (sont) choisi(s) parmi les agents tensioactifs suivants : sodium lauryl sulfate, un ester de polysorbitanne polyoxyéthyléné, tel que les esters monooléate, monolaurate, monopalmitate, monostéarate, dioctylsulfosuccinate de sodium (DOSS) et lécithine.

12. Procédé selon l'une des revendications 1 à 11, **caractérisé en ce que** le ou les agent(s) anti-statique(s) est (sont) choisi(s) parmi la silice colloïdale, le silicate de magnésium, le talc, le silicate de calcium et le phosphate de calcium tribasique.

13. Procédé selon l'une des revendications 1 à 12, **caractérisé en ce que** le ou les agent(s) diluant(s) est (sont) choisi(s) parmi le carbonate ou bicarbonate de calcium ou de sodium, le sucrose, le mannitol, le xylitol, le sorbitol, le lactose, le maltitol, le glucose, la poudre de cellulose ou cellulose microcristalline, l'amidon et ses dérivés, le phosphate de calcium dibasique, le phosphate de calcium tribasique, le sulfate de calcium, les dextrates, les dextrines, les excipients de dextrose, le fructose, le kaolin, le lactitol.

14. Procédé selon l'une des revendications 1 à 13, **caractérisé en ce que** le ou les agent(s) désintégrant(s) est (sont) choisi(s) parmi le glycolate d'amidon sodique, la croscarmellose sodique, la polyvinylpyrrolidone réticulée, la carboxymethylcellulose de sodium et la carboxyméthylcellulose de calcium et l'hydroxypropylcellulose faiblement substituée.

15. Procédé selon l'une des revendications 1 à 14, **caractérisé en ce que** le ou les agent(s) lubrifiant(s) est (sont) choisi(s) parmi le stéarate de magnésium, le stéarate de calcium et le talc.

16. Procédé selon l'une des revendications 1 à 15, **caractérisé en ce qu** il comprend une étape supplémentaire (g) de pelliculage du comprimé obtenu à l'étape (f).

17. Comprimé de fénofibrate ou de l'un de ses dérivés, éventuellement en association avec un second principe actif, susceptible d'être obtenu par le procédé de la revendication 1 **caractérisé en ce qu'**il comprend :
(a) une phase interne constituée de :
(i) de 20 % à 50 % en poids de fénofibrate ou de l'un de ses dérivés et éventuellement un second principe actif, sous la forme d'un micronisat avec au moins un agent tensioactif solide, ledit micronisat comprenant (i) de 91 % à 99 % en poids de fénofibrate ou de l'un de ses dérivés, éventuellement en association avec le second principe actif, et (ii) de 1 % à 9 % en poids dudit ou desdits agent(s) tensioactif(s) solide(s);
(ii) de 0,2 % à 2 % en poids d'au moins un agent antistatique ;
(iii) de 40 % à 80 % en poids d'au moins un agent diluant, ;
(iv) de 1 % à 20 % en poids d'au moins un agent désintégrant; et
(v) de 0,1 % à 1 % d'au moins un agent lubrifiant ; par rapport au poids total dudit comprimé ; et
(b) une phase externe comprenant de 0,1 % à 2 % d'au moins un agent lubrifiant, par rapport au poids total dudit comprimé.

18. Comprimé selon la revendication 17, **caractérisé en ce que** la phase externe est recouverte d'un vernis protecteur, de préférence un vernis protecteur à base d'un polymère hydrodispersible.

19. Comprimé selon l'une des revendications 17 ou 18, dans lequel le fénofibrate ou son dérivé est en association avec un second principe actif.

20. Comprimé selon la revendication 19, **caractérisé en ce que** le second principe actif est choisi parmi la metformine, la cobalamine, l'acide folique, la bétaïne, la N -acétylcystéine, la vitamine E et un inhibiteur de HGMCoA.

## Claims

1. Method for the manufacture of a pharmaceutical composition containing the active ingredient fenofibrate or one of its derivatives, optionally in the form of a combination of fenofibrate or its derivative with a second active ingredient, in the form of tablets, **characterized in that** it comprises the following steps :
(a) prepare a mixture of fenofibrate or one of its derivatives, or of the combination with a second active ingredient, and at least one solid surfactant, in a ratio of (i) 91% to 99% by weight of fenofibrate or its derivative and (ii) 1% to 9% by weight of the said solid surfactant(s);
(b) micronize the mixture of fenofibrate or one of its derivatives, optionally in the form of a combination with a second active ingredient, and surfactant(s) obtained in step (a) in order to obtain a micronizate of fenofibrate or of the combination with the second active ingredient, and the surfactant(s);
(c) add at least one anti-static agent to the co-micronizate prepared in step (b);
(d) add to the mixture obtained in step (c) at least one diluent, at least one disintegrant and at least one lubricant in order to obtain a solid mixture corresponding to the internal phase of the tablet;
(e) compress the solid mixture obtained in step (d) through a dry granulation step, in order to obtain the final internal phase of the tablet;
(f) mix the internal phase of the tablet prepared in step (e) with an external phase comprising at least one lubricant, then carry out a compression of the composition in order to obtain the pharmaceutical composition containing fenofibrate or one of its derivatives in the form of tablets.

2. Method according to claim 1, wherein in step (a), the mixture of fenofibrate or one of its derivatives, optionally in the form of a combination with a second active ingredient, and surfactant(s) comprises a ratio of (i) 95% to 98% by weight of fenofibrate or the combination and (ii) 2% to 5% by weight of surfactant (s) .

3. Method according to anyone of claims 1 or 2, wherein in step (b), the co-micronizate consists of particles possessing a size included between 0.1 and 20 µm.

4. Method according to anyone of claims 1 to 3, wherein in step (c), the anti-static agent(s) is/are added in an amount of 0.1% to 5% by weight, preferably of 0.2% to 2% by weight with respect to the total weight of the composition.

5. Method according to anyone of claims 1 to 4, wherein in step (d), the diluent(s) is/are added in an amount of 40% to 80% by weight, preferably of 50% to 75% by weight with respect to the total weight of the composition.

6. Method according to anyone of claims 1 to 5, wherein in step (d), the disintegrant(s) is/are added in an amount of 1% to 20% by weight, preferably of 3% to 6% by weight with respect to the total weight of the composition.

7. Method according to anyone of claims 1 to 6, wherein in step (d), the lubricant(s) is/are added in an amount of 0.1% to 2% by weight, preferably of 0.2% to 1% by weight with respect to the total weight of the composition.

8. Method according to anyone of claims 1 to 7, wherein the fenofibrate or its derivative is present in an amount of 20% to 50% by weight, with respect to the total weight of the composition.

9. Method according to claim 8, wherein the fenofibrate or its derivative is combined with a second active ingredient.

10. Method according to claim 9, wherein said second active ingredient is selected from metformin, cobalamine, folic acid, betaine, N-acetylcysteine, vitamin E and an inhibitor of HGMCoA.

11. Method according to anyone of claims 1 to 10, wherein the surfactant(s) is/are selected from the following surfactants: sodium lauryl sulfate, a polyoxyethylenated ester of polysorbitan, such as the monooleate, monolaurate, monopalmitate, monostearate esters, sodium dioctylsulfosuccinate (DOSS) and lecithin.

12. Method according to anyone of claims 1 to 11, wherein the anti-static agent(s) is/are selected from colloidal silica, magnesium silicate, talc, calcium silicate and tribasic calcium phosphate.

13. Method according to anyone of claims 1 to 12, wherein the diluent(s) is/are selected from calcium or sodium carbonate or bicarbonate, sucrose, mannitol, xylitol, sorbitol, lactose, maltitol, glucose, cellulose powder or microcrystalline cellulose, starch and its derivatives, dibasic calcium phosphate, tribasic calcium phosphate, calcium sulfate, dextrates, dextrins, dextrose excipients, fructose, kaolin, lactitol.

14. Method according to anyone of claims 1 to 13, wherein the disintegrant(s) is/are selected from sodium starch glycollate, sodium croscarmellose, crosslinked polyvinylpyrrolidone, sodium carboxymethylcellulose and calcium carboxymethylcellulose and lightly substituted hydroxypropylcellulose.

15. Method according to anyone of claims 1 to 14, wherein the lubricant(s) is/are selected from magnesium stearate, calcium stearate and talc.

16. Method according to anyone of claims 1 to 15, wherein it comprises an additional film-coating step (g) of the tablet obtained in step (f).

17. Tablet of fenofibrate or one of its derivatives, optionally in combination with a second active ingredient obtainable by the method of claim 1, wherein said tablet comprises :
(a) an internal phase consisting of :
(i) 20% to 50% by weight of fenofibrate or one of its derivatives and optionally a second active ingredient, in the form of a micronizate with at least one solid surfactant, said micronizate comprising (i) from 91% to 99% by weight of fenofibrate or one of its derivatives, optionally in combination with the second active ingredient, and (ii) from 1% to 9% by weight of the said solid surfactant(s) ;
(ii) from 0.2% to 2% by weight of at least one anti-static agent ;
(iii) from 40% to 80% by weight of at least one diluent ;
(iv) from 1% to 20% by weight of at least one disintegrant ; and
(v) from 0.1% to 1% of at least one lubricant ;
with respect to the total weight of the said tablet; and
(b) an external phase comprising from 0.1% to 2% at least one lubricant with respect to the total weight of the said tablet.

18. Tablet according to claim 17, wherein the external phase is covered with a protective varnish, preferably a protective varnish based on a hydrodispersible polymer.

19. Tablet according to anyone of claims 17 or 18, in which the fenofibrate or its derivative is combined with a second active ingredient.

20. Tablet according to claim 19, wherein the second active ingredient is selected from mefformin, cobalamine, folic acid, betaine, N-acetylcysteine, vitamin E and an inhibitor of HGMCoA.

## Patentansprüche

1. Verfahren zur Herstellung einer pharmazeutischen Zusammensetzung, die das aktive Prinzip Fenofibrat oder eines seiner Derivate, gegebenenfalls in Form einer Kombination des Fenofibrats oder seines Derivats mit einem zweiten aktiven Prinzip enthält, in Tablettenform, **dadurch gekennzeichnet, dass** es die folgenden Schritte umfasst:
(a) Herstellen eines Gemisches von Fenofibrat oder einem seiner Derivate oder der Kombination mit einem zweiten aktiven Prinzip und wenigstens einem festen Tensid in einem Anteil von (i) 91 bis 99 Gew.-% Fenofibrat oder seines Derivats und (ii) 1 bis 9 Gew.-% des festen Tensids oder der Tenside;
(b) Mikronisieren des in Schritt (a) erhaltenen Gemisches von Fenofibrat oder eines seiner Derivate, gegebenenfalls in Form einer Kombination mit einem zweiten aktiven Prinzip, und eines Tensids (Tensiden) unter Erhalten eines Mikronisats des Fenofibrats oder der Kombination mit dem zweiten aktiven Prinzip und des Tensids oder der Tenside;
(c) Zusetzen wenigstens eines Antistatikums zu dem in Schritt (b) hergestellten Comikronisat;
(d) Zusetzen wenigstens eines Verdünnungsmittels, wenigstens eines Zerfallhilfsmittels und wenigstens eines Gleitmittels zu dem in Schritt (c) erhaltenen Gemisch unter Erhalten eines der inneren Phase der Tablette entsprechenden festen Gemisches;
(e) Verpressen des in Schritt (d) erhaltenen festen Gemisches in einem Trockengranulierungsschritt unter Erhalten der endgültigen inneren Phase der Tablette;
(f) Mischen der in Schritt (e) hergestellten inneren Phase der Tablette mit einer äußeren Phase, die wenigstens ein Gleitmittel umfasst, und anschließend Durchführen eines Verpressens der Zusammensetzung unter Erhalten einer pharmazeutischen Zusammensetzung, die das Fenofibrat oder eines seiner Derivate in Tablettenform enthält.

2. Verfahren gemäß Anspruch 1, **dadurch gekennzeichnet, dass** in Schritt (a) das Gemisch von Fenofibrat oder einem seiner Derivate, gegebenenfalls in Form einer Kombination mit einem zweiten aktiven Prinzip, und einem Tensid (Tensiden) einen Anteil von (i) 95 bis 98 Gew.-% Fenofibrat oder der Kombination und (ii) 2 bis 5 Gew.-% Tensid(e) umfasst.

3. Verfahren gemäß einem der Ansprüche 1 und 2, **dadurch gekennzeichnet, dass** in Schritt (b) das Comikronisat eine Größe zwischen 0,1 und 20 µm besitzende Teilchen umfasst.

4. Verfahren gemäß einem der Ansprüche 1 bis 3, **dadurch gekennzeichnet, dass** in Schritt (c) das Antistatikum oder die Antistatika in einer Menge von 0,1 bis 5 Gew.-%, vorzugsweise von 0,2 bis 2 Gew.-% bezogen auf das Gesamtgewicht der Zusammensetzung zugesetzt wird (werden).

5. Verfahren gemäß einem der Ansprüche 1 bis 4, **dadurch gekennzeichnet, dass** in Schritt (d) das oder die Verdünnungsmittel in einer Menge von 40 bis 80 Gew.-%, vorzugsweise zwischen 50 und 75 Gew.-% bezogen auf das Gesamtgewicht der Zusammensetzung zugesetzt wird (werden).

6. Verfahren gemäß einem der Ansprüche 1 bis 5, **dadurch gekennzeichnet, dass** in Schritt (d) das oder die Zerfallhilfsmittel in einer Menge von 1 bis 20 Gew.-%, vorzugsweise von 3 bis 6 Gew.-% bezogen auf das Gesamtgewicht der Zusammensetzung zugesetzt wird (werden).

7. Verfahren gemäß einem der Ansprüche 1 bis 6, **dadurch gekennzeichnet, dass** in Schritt (d) das oder die Gleitmittel in einer Menge von 0,1 bis 2 Gew.-%, vorzugsweise von 0,2 bis 1 Gew.-% bezogen auf das Gesamtgewicht der Zusammensetzung zugesetzt wird (werden).

8. Verfahren gemäß einem der Ansprüche 1 bis 7, **dadurch gekennzeichnet, dass** das Fenofibrat oder sein Derivat in einer Menge von 20 bis 50 Gew.-% bezogen auf das Gesamtgewicht der Zusammensetzung vorliegt.

9. Verfahren gemäß Anspruch 8, **dadurch gekennzeichnet, dass** das Fenofibrat oder sein Derivat mit einem zweiten aktiven Prinzip kombiniert ist.

10. Verfahren gemäß Anspruch 9, **dadurch gekennzeichnet, dass** das zweite aktive Prinzip aus Metformin, Cobalamin, Folsäure, Betain, N-Acetylcystein, Vitamin E und einem HMG-CoA-Hemmer ausgewählt ist.

11. Verfahren gemäß einem der Ansprüche 1 bis 10, **dadurch gekennzeichnet, dass** das oder die Tensid(e) aus den folgenden Tensiden ausgewählt ist (sind): Natriumlaurylsulfat, einem polyoxyethylenierten Polysorbitanester wie etwa Monooleat-, Monolaurat-, Monopalmitat- und Monostearatestern, Natriumdioctylsulfosuccinat (DOSS) und Lecithin.

12. Verfahren gemäß einem der Ansprüche 1 bis 11, **dadurch gekennzeichnet, dass** das Antistatikum oder die Antistatika aus kolloidalem Siliziumoxid, Magnesiumsilikat, Talkum, Calciumsilikat und tribasischem Calciumphosphat ausgewählt ist (sind).

13. Verfahren gemäß einem der Ansprüche 1 bis 12, **dadurch gekennzeichnet, dass** das oder die Verdünnungsmittel aus Calcium- oder Natriumcarbonat oder -bicarbonat, Sucrose, Mannit, Xylit, Sorbit, Lactose, Maltit, Glucose, Cellulosepulver oder mikrokristalliner Cellulose, Amidon und seinen Derivaten, dibasischem Calciumphosphat, tribasischem Calciumphosphat, Calciumsulfat, Dextraten, Dextrinen, Dextroseexzipienten, Fructose, Kaolin und Lactit ausgewählt ist (sind).

14. Verfahren gemäß einem der Ansprüche 1 bis 13, **dadurch gekennzeichnet, dass** das oder die Zerfallhilfsmittel aus Natriumamidonglykolat, Natriumcroscarmellose, vernetztem Polyvinylpyrrolidon, Natriumcarboxymethylcellulose und Calciumcarboxymethylcellulose und niedrigsubstituierter Hydroxypropylcellulose ausgewählt ist (sind).

15. Verfahren gemäß einem der Ansprüche 1 bis 14, **dadurch gekennzeichnet, dass** das oder die Gleitmittel aus Magnesiumstearat, Calciumstearat und Talkum ausgewählt ist (sind).

16. Verfahren gemäß einem der Ansprüche 1 bis 15, **dadurch gekennzeichnet, dass** es einen zusätzlichen Schritt (g) der Beschichtung der in Schritt (f) erhaltenen Tablette umfasst.

17. Tablette aus Fenofibrat oder einem seiner Derivate, gegebenenfalls in Kombination mit einem zweiten aktiven Prinzip, die durch das Verfahren des Anspruchs 1 erhalten werden kann, **dadurch gekennzeichnet, dass** sie Folgendes umfasst:
(a) eine innere Phase bestehend aus:
(i) 20 bis 50 Gew.-% Fenofibrat oder eines seiner Derivate und gegebenenfalls einem zweiten aktiven Prinzip in Form eines Mikronisats mit wenigstens einem festen Tensid, wobei das Mikronisat (i) 91 bis 99 Gew.-% Fenofibrat oder eines seiner Derivate, gegebenenfalls in Kombination mit dem zweiten aktiven Prinzip, und (ii) 1 bis 9 Gew.-% des oder der festen Tenside umfasst;
(ii) 0,2 bis 2 Gew.-% wenigstens eines Antistatikums;
(iii) 40 bis 80 Gew.-% wenigstens eines Verdünnungsmittels;
(iv) 1 bis 20 Gew.-% wenigstens eines Zerfallhilfsmittels und
(v) 0,1 bis 1 % wenigstens eines Gleitmittels
bezogen auf das Gesamtgewicht der Tablette und
(b) eine äußere Phase, die 0,1 bis 2 % wenigstens eines Gleitmittels bezogen auf das Gesamtgewicht der Tablette umfasst.

18. Tablette gemäß Anspruch 17, **dadurch gekennzeichnet, dass** die äußere Phase mit einem Schutzlack, vorzugsweise einem Schutzlack auf der Grundlage eines wasserdispergierbaren Polymers überzogen ist

19. Tablette gemäß einem der Ansprüche 17 oder 18, bei der das Fenofibrat oder sein Derivat mit einem zweiten aktiven Prinzip kombiniert ist.

20. Tablette gemäß Anspruch 19, **dadurch gekennzeichnet, dass** das zweite aktive Prinzip aus Metformin, Cobalamin, Folsäure, Betain, N-Acetylcystein, Vitamin E und einem HMG-CoA-Hemmer ausgewählt ist.
